(19) Europäisches Patentamt
European Patent Office
Office européen des brevets

(11) Publication number: **0 412 710 B1**

# (12) EUROPEAN PATENT SPECIFICATION

(45) Date of publication of patent specification: **19.07.95**

(51) Int. Cl.$^6$: **A61K 7/48**, A61K 7/06, A61K 7/08

(21) Application number: **90308435.8**

(22) Date of filing: **31.07.90**

## (54) Vehicle systems for use in cosmetic compositions.

(30) Priority: **07.08.89 US 390330**
**01.05.90 US 517289**
**16.07.90 US 551120**

(43) Date of publication of application:
**13.02.91 Bulletin 91/07**

(45) Publication of the grant of the patent:
**19.07.95 Bulletin 95/29**

(84) Designated Contracting States:
**AT BE CH DE DK ES FR GB GR IT LI LU NL SE**

(56) References cited:
**EP-A- 0 189 935** **EP-A- 0 190 010**
**EP-A- 0 213 527** **EP-A- 0 229 400**
**US-A- 4 834 972** **US-A- 4 894 224**

**CHEMICAL ABSTRACTS, vol. 103, no. 4, 29 July 1985, Columbus, Ohio, US; abstractno. 27060B,**

(73) Proprietor: **THE PROCTER & GAMBLE COMPANY**
**One Procter & Gamble Plaza**
**Cincinnati,**
**Ohio 45202 (US)**

(72) Inventor: **Bolich, Raymond Edward, Jr.**
**7201 Striker Rd.**
**Maineville, OH 45039 (US)**
Inventor: **Norton, Michael James**
**3628 Michigan Ave.**
**Cincinnati, OH 45208 (US)**
Inventor: **Russell, Glen David**
**6099 Donna Jay Dr.**
**Loveland, OH 45140 (US)**

(74) Representative: **Brooks, Maxim Courtney et al**
**Procter & Gamble Limited**
**Whitley Road**
**Longbenton**
**Newcastle-upon-Tyne NE12 9TS (GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel vehicle systems, and cosmetic compositions formulated therewith, based on particular nonionic long chain alkylated water-soluble polymer derivatives and water-insoluble surfactants at certain critical levels, dispersed in a compatible solvent. A particularly useful application of the present invention is in hair care compositions, especially rinse-off hair conditioning compositions.

Typical hair conditioning products have a particular thick rheology that is desirable for such products. These products are based on the combination of a surfactant, which is generally a quaternary ammonium compound, and a fatty alcohol. This combination results in a gel-network structure which provides the composition with a thick rheology. However, while such compositions deliver conditioning benefits to the hair, such compositions also deposit on hair making hair look and feel dirty.

Alternative thickening systems have been used in hair care compositions, but none have been found to date which provide this same desirable rheology. Though hair care products thickened with polymer thickeners can be made to have a thick rheology, these products generally are characterized by an undesirable "slimy" feel and do not hold their poured shape.

Nonionic water-soluble cellulose ethers are employed in a variety of applications, including hair care compositions. Widely used, commercially-available nonionic cellulose ethers include methyl cellulose, hydroxy propyl methyl cellulose, hydroxyethyl cellulose, hydroxypropyl cellulose and ethyl hydroxyethyl cellulose.

Better thickening efficiency is realized with higher molecular weight cellulose ethers. However, production of such materials is difficult and expensive. Though crosslinking of these polymers is an alternative means to achieve high viscosity solutions, good crosslinking techniques are not known. Of course, high concentrations of polymers will also provide high viscosity but such an approach is inefficient and impractical, particularly due to the high expense involved. Furthermore, use of highly crosslinked polymers or high levels of polymeric thickeners may result in a vehicle system that is too elastic for the present uses.

Alternative water-soluble polymeric thickeners sometimes used to thicken hair care compositions are natural polysaccharides such as guar gum, xanthan gum and locust bean gum.

A number of references teach the use of nonionic cellulose ethers and water-soluble gums for thickening hair care compositions. See for example, U.S. Patent 4,557,928, Glover, issued December 10, 1985, teaching a hair conditioner comprising a suspension system which consists of one of glucan gum, guar gum, and hydroxyethylcellulose; and U.S. Patent 4,581,230, Grollier et al., issued April 8, 1986, which teaches cosmetic compositions for treating hair which comprise as thickening agents hydroxyethylcellulose, or water-soluble vegetable thickening agents, such as guar gum. Japanese Patent Publication 61-053211, published March 7, 1986, discloses a hair colorant containing an aromatic alcohol, xanthan gum, and hydroxyethylcellulose.

Certain cellulose ethers have been disclosed in U.S. Patent 4,228,277, Landoll, issued October 14, 1980, which are relatively low molecular weight but which are capable of producing highly viscous aqueous solutions in practical concentrations. These materials are nonionic cellulose ethers having a sufficient degree of nonionic substitution selected from the group consisting of methyl, hydroxyethyl, and hydroxypropyl to cause them to be water-soluble and which are further substituted with a hydrocarbon radical having from 10 to 24 carbon atoms in an amount between 0.2 weight percent and the amount which renders said cellulose ether less than 1%, by weight, soluble in water. The cellulose ether to be modified is preferably one of low to medium molecular weight; i.e., less than 800,000 and preferably between 20,000 and 700,000 (75 to 2500 D.P.).

These modified cellulose ethers have been disclosed for use in a variety of composition types. Landoll ('277) teaches the use of these materials in shampoo formulations. Hercules trade literature teaches the use of these materials in shampoos, liquid soaps, and lotions. U.S. Patent 4,683,004 discloses the use of these materials in mousse compositions for the hair. U.S. Patent 4,485,089 teaches dentifrice compositions containing these materials.

These materials have now been found to provide a rheology very much like the desirable gel-network structure of typical hair conditioners (without the slimy feel associated with most polymeric thickeners), when they are combined with surfactants at certain critical levels.

Hence, it is an object of the present invention to provide a vehicle system for a hair care and other cosmetic composition which provides a gel-network-like structure to the composition but which is not based on a typical quaternary ammonium compound/fatty alcohol gel-network thickening system.

It is also an object of the present invention to provide a vehicle system for a hair care and other cosmetic compositions which allows for dispersion of a wide variety of active hair or skin care components therein.

It is also an object of the present invention to provide a vehicle system for hair care and other cosmetic compositions which will maximize deposition of the active hair or skin care component contained therein onto the hair or skin while minimizing deposition of the vehicle system components.

These and other objects will become readily apparent from the detailed description which follows.

The present invention relates to unique vehicle systems for use in cosmetic compositions which are polymer-based but which provide a rheology to the cosmetic compositions which mimics gel-network systems. These vehicle systems are based on a two-component thickening system. More specifically, the cosmetic compositions of the present invention comprise:

(a) from 80% to 100%, preferably from 80% to 99.9%, of a vehicle system which comprises:

(A) from 0.1% to 10% by weight of the cosmetic composition of a hydrophobically modified nonionic water-soluble polymer which comprises a water-soluble polymer backbone and hydrophobic groups selected from the group consisting of $C_8$-$C_{22}$ alkyl, aryl alkyl, alkyl aryl groups and mixtures thereof; wherein the ratio of hydrophilic portion to hydrophobic portion of the polymer is from 10:1 to 1000:1; preferably the hydrophobically modified nonionic water-soluble polymer comprises a nonionic cellulose ether having a sufficient degree of nonionic substitution selected from the group consisting of methyl, hydroxyethyl, and hydroxypropyl to cause it to be water-soluble and being further substituted with a long chain alkyl radical having 10 to 24 carbon atoms in an amount between 0.2 weight percent and the amount which renders said cellulose ether less than 1% by weight soluble in water;

(B) from 0.02% to 5.0% by weight of the cosmetic composition of water-insoluble surfactant having a molecular weight less than 20,000;

(C) from 65% to 99% by weight of the cosmetic composition of a compatible solvent; and

(b) from 0 to 20%, preferably from 0.1% to 20%, of an active cosmetic component;

wherein compositions comprising said vehicle system comprise no more than 1.0%, preferably no more than 0.5%, of water-soluble surfactant materials.

The vehicle system provides a rheology to the cosmetic compositions formulated therewith, that is preferably characterized by a shear stress of from 0 to 50 pascal over a shear rate range of from 0.04 $sec^{-1}$ to 25 $sec^{-1}$.

These vehicle systems are particularly useful in hair care compositions especially rinse-off hair conditioners. Most preferably, the hair care compositions formulated with these unique vehicle systems comprise no more than 1% of fatty alcohol materials.

The essential as well as optional components of the present compositions are described below.

## Primary Thickener

The vehicle systems of the present invention contain, as an essential component, a primary thickening material. The primary thickening material is a hydrophobically modified nonionic water-soluble polymer. By "hydrophobically modified nonionic water-soluble polymer" is meant a nonionic water-soluble polymer which has been modified by the substitution with a sufficient amount of hydrophobic groups to make the polymer less soluble in water. Hence, the polymer backbone of the primary thickener can be essentially any water-soluble polymer. The hydrophobic groups can be $C_8$ to $C_{22}$ alkyl, aryl alkyl, alkyl aryl groups and mixtures thereof. The degree of hydrophobic substitution on the polymer backbone should be from 0.10% to 1.0%, depending on the particular polymer backbone. More generally, the ratio of hydrophilic portion to hydrophobic portion of the polymer is from 10:1 to 1000:1.

A number of existing patents disclose nonionic polymer materials which meet the above requirements and which are useful in the present invention. U.S. Patent 4,496,708 teaches water-soluble polyurethanes having hydrophilic polyether backbones and pendant monovalent hydrophobic groups to result in a hydrophilic/lipophilic balance of between 14 and 19.5. U.S. Patent 4,426,485 discloses a water-soluble thermo-plastic organic polymer having segments of bunched monovalent hydrophobic groups. U.S. Patent 4,415,701 discloses copolymers containing a monoepoxide and a dioxolane.

The most preferred primary thickener materials for use in the present invention are disclosed in U.S. Patent 4,228,277. The materials disclosed therein are thickeners comprising a nonionic long chain alkylated cellulose ether.

The cellulose ethers have a sufficient degree of nonionic substitution selected from the group consisting of methyl, hydroxyethyl and hydroxypropyl to cause them to be water-soluble. The cellulose ethers are further substituted with a hydrocarbon radical having 10 to 24 carbon atoms in an amount between 0.2 weight percent and the amount which renders said cellulose ether less than 1%, by weight, soluble in water. The cellulose ether to be modified is preferably one of low to medium molecular weight, i.e., less than 800,000 and preferably between 20,000 and 700,000 (75 to 2500 D.P.).

The Landoll patent teaches that any nonionic water-soluble cellulose ether can be employed as the cellulose ether substrate. Thus, e.g., hydroxyethyl cellulose, hydroxypropyl cellulose, methyl cellulose, hydroxypropyl methyl cellulose, ethyl hydroxyethyl cellulose, and methyl hydroxyethyl cellulose can all be modified. The amount of nonionic substituent such as methyl, hydroxyethyl or hydroxypropyl is taught not to be critical so long as there is an amount sufficient to assure that the ether is water-soluble.

The preferred cellulose ether substrate is hydroxyethyl cellulose (HEC) of 50,000 to 700,000 molecular weight. Hydroxyethyl cellulose of this molecular weight level is the most hydrophilic of the materials contemplated. It can thus be modified to a greater extent than can other water-soluble cellulose ether substrates before insolubility is achieved. Accordingly, control of the modification process and control of the properties of the modified product can be more precise with this substrate. Hydrophilicity of the most commonly used nonionic cellulose ethers varies in the general direction: hydroxyethyl → hydroxypropyl → hydroxypropyl methyl → methyl.

The long chain alkyl modifier can be attached to the cellulose ether substrate via an ether, ester or urethane linkage. The ether linkage is preferred.

Although the materials taught in Landoll are referred to as being "long chain alkyl group modified", it will be recognized that except in the case where modification is effected with an alkyl halide, the modifier is not a simple long chain alkyl group. The group is actually an alphahydroxyalkyl radical in the case of an epoxide, a urethane radical in the case of an isocyanate, or an acyl radical in the case of an acid or acyl chloride. Nonetheless, the terminology "long chain alkyl group" is used since the size and effect of the hydrocarbon portion of the modifying molecule completely obscure any noticeable effect from the connecting group. Properties are not significantly different from those of the product modified with the simple long chain alkyl group.

Methods for making these modified cellulose ethers are taught in Landoll ('277) at column 2, lines 36-65.

These materials have been found to be particularly desirable for use in the vehicle systems of the cosmetic compositions of the present invention. The materials are able to stabilize suspensions of dispersed phases, and when used with the additional components in the vehicle systems of the present invention, they produce rheologically thick products which lack the slimy feel characteristic of most polymeric thickeners.

One commercially available material which meets these requirements is NATROSOL PLUS Grade 330, a hydrophobically modified hydroxyethylcellulose available from Aqualon Company, Wilmington, Delaware. This material has a $C_{16}$ alkyl substitution of from 0.4% to 0.8% by weight. The hydroxyethyl molar substitution for this material is from 3.0 to 3.7. The average molecular weight for the water-soluble cellulose prior to modification is approximately 300,000.

Another material of this type is sold under the trade name NATROSOL PLUS CS Grade D-67, by Aqualon Company, Wilmington, Delaware. This material has a $C_{16}$ alkyl substitution of from 0.50% to 0.95%, by weight. The hydroxyethyl molar substitution for this material is from 2.3 to 3.7. The average molecular weight for the water soluble cellulose prior to modification is approximately 700,000.

The primary thickener component is present in the cosmetic compositions of the present invention at from 0.1% to 10.0%, preferably from 0.2% to 5.0%.

It is important that the primary thickener be well-hydrated and dispersed in the compositions of the present invention.

Water-Insoluble Surfactant

The present vehicle systems further comprise, as a second essential component, a water-insoluble surfactant having a molecular weight of less than 20,000. By "water-insoluble surfactant" is meant surfactant materials which do not form clear isotropic solutions when dissolved in water at greater than 0.2 weight percent at ambient conditions.

Nonlimiting examples of water-insoluble surfactants which can be used in the vehicle systems of the compositions of the present invention can be selected from water-insoluble anionic, nonionic, cationic, zwitterionic and amphoteric surfactants.

Synthetic anionic surfactants include alkyl and alkyl ether sulfates. These materials have the respective formulae $ROSO_3M$ and $RO(C_2H_4O)_xSO_3M$, wherein R is alkyl or alkenyl of from about 10 to about 20 carbon atoms, x is 1 to 10, and M is a water-soluble cation such as ammonium, sodium, potassium and triethanolamine. The alkyl ether sulfates useful in the present invention are condensation products of ethylene oxide and monohydric alcohols having from 10 to 20 carbon atoms. Preferably, R has from 14 to 20 carbon atoms in both the alkyl and alkyl ether sulfates. The alcohols can be derived from fats, e.g.,

coconut oil or tallow, or can be synthetic. Stearyl alcohol and straight chain alcohols derived from tallow oil are preferred herein. Such alcohols are reacted with about 1 to about 10, and especially about 3, molar proportions of ethylene oxide and the resulting mixture of molecular species, having, for example, an average of 3 moles of ethylene oxide per mole of alcohol, is sulfated and neutralized.

Specific examples of alkyl ether sulfates which can be used in the present invention are sodium tallow alkyl diethylene glycol ether sulfate; and sodium tallow alkyl sulfate.

Another suitable class of anionic surfactants are the salts of the organic, sulfuric acid reaction products of the general formula:

$R_1$ - $SO_3$ - M

wherein $R_1$ is chosen from the group consisting of a straight or branched chain, saturated aliphatic hydrocarbon radical having from 8 to 24, preferably 18 to 22, carbon atoms; and M is a cation. Important examples are the salts of an organic sulfuric acid reaction product of a hydrocarbon of the methane series, including iso-, neo-, ineso-, and n-paraffins, having 8 to 24 carbon atoms, preferably 12 to 18 carbon atoms and a sulfonating agent, e.g., $SO_3$, $H_2SO_4$, oleum, obtained according to known sulfonation methods, including bleaching and hydrolysis. Preferred are alkali metal and ammonium sulfonated $C_{12-18}$ n-paraffins.

Additional examples of anionic synthetic surfactants which can be used in the present invention are the reaction products of fatty acids esterified with isethionic acid and neutralized with sodium hydroxide where, for example, the fatty acids are derived from tallow oil; sodium or potassium salts of fatty acid amides of methyl tauride in which the fatty acids, for example, are derived from tallow oil. Other anionic synthetic surfactants of this variety are set forth in U.S. Patents 2,486,921; 2,486,922; and 2,396,278.

Still other anionic synthetic surfactants include the class designated as succinamates. This class includes such surface active agents as disodium N-octadecylsulfosuccinamate; tetrasodium N-(1,2-dicarboxyethyl)-N-octadecylsulfosuccinamate; dioctyl esters of sodium sulfosuccinic acid.

Other suitable anionic surfactants utilizable herein are olefin sulfonates having 12 to 24 carbon atoms. The term "olefin sulfonates" is used herein to mean compounds which can be produced by the sulfonation of α-olefins by means of uncomplexed sulfur trioxide, followed by neutralization of the acid reaction mixture in conditions such that any sultones which have been formed in the reaction are hydrolyzed to give the corresponding hydroxy-alkanesulfonates. The sulfur trioxide can be liquid or gaseous, and is usually, but not necessarily, diluted by inert diluents, for example by liquid $SO_2$, chlorinated hydrocarbons, etc., when used in the liquid form, or by air, nitrogen, gaseous $SO_2$, etc., when used in the gaseous form.

The α-olefins from which the olefin sulfonates are derived are mono-olefins having 12 to 24 carbon atoms, preferably 14 to 24 carbon atoms. Preferably, they are straight chain olefins. Examples of suitable 1-olefins include 1-dodecene; 1-tetradecene; 1-hexadecene; 1-octadecene; 1-eicosene and 1-tetracosene.

In addition to the true alkene sulfonates and a proportion of hydroxy-alkanesulfonates, the olefin sulfonates can contain minor amounts of other materials, such as alkene disulfonates depending upon the reaction conditions, proportion of reactants, the nature of the starting olefins and impurities in the olefin stock and side reactions during the sulfonation process.

A specific α-olefin sulfonate mixture of the above type is described more fully in the U.S. Patent 3,332,880.

Another class of anionic organic surfactants are the β-alkyloxy alkane sulfonates. These compounds have the following formula:

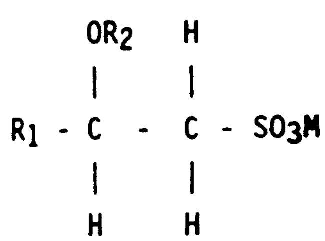

where $R_1$ is a straight chain alkyl group having from 6 to 20 carbon atoms, $R_2$ is a lower alkyl group having from about 1 (preferred) to 3 carbon atoms, and M is a water-soluble cation as hereinbefore described.

Many additional nonsoap synthetic anionic surfactants are described in McCutcheon's, Detergents and Emulsifiers, 1984 Annual, published by Allured Publishing Corporation. Also U.S. Patent 3,929,678 discloses many other anionic as well as other surfactant types.

Nonionic surfactants can be broadly defined as compounds containing a hydrophobic moiety and a nonionic hydrophilic moiety. Examples of the hydrophobic moiety can be alkyl, alkyl aromatic, dialkyl siloxane, polyoxyalkylene, and fluoro-substituted alkyls. Examples of hydrophilic moieties are polyoxyalkylenes, phosphine oxides, sulfoxides, amine oxides, and amides. Examples of preferred classes of nonionic surfactants are:

1. The polyethylene oxide condensates of alkyl phenols, e.g., the condensation products of alkyl phenols having an alkyl group containing from 6 to 12 carbon atoms in either a straight chain or branched chain configuration, with ethylene oxide, the said ethylene oxide being present in amounts equal to from 2 to 6 moles of ethylene oxide per mole of alkyl phenol. The alkyl substituent in such compounds may be derived from polymerized propylene, diisobutylene, octane, or nonane, for example.

2. Those derived from the condensation of ethylene oxide with the product resulting from the reaction of propylene oxide and ethylene diamine products which may be varied in composition depending upon the balance between the hydrophobic and hydrophilic elements which is desired. For example, compounds containing from 10% to 40% polyoxyethylene by weight and having a molecular weight of from 500 to 4,000 resulting from the reaction of ethylene oxide groups with a hydrophobic base constituted of the reaction product of ethylene diamine and excess propylene oxide, said base having a molecular weight of the order of about 2,500 to about 10,000, are satisfactory.

3. The condensation product of aliphatic alcohols having from 8 to 20 carbon atoms, in either straight chain or branched chain configuration, with ethylene oxide, e.g., a tallow alcohol ethylene oxide condensate having from 2 to 10 moles of ethylene oxide per mole of tallow alcohol, the tallow alcohol fraction having from 16 to 18 carbon atoms.

4. Long chain tertiary amine oxides corresponding to the following general formula:

$$R_1R_2R_3N \rightarrow O$$

wherein $R_1$ contains an alkyl, alkenyl or monohydroxy alkyl radical of from 12 to 22 carbon atoms, from 0 to 10 ethylene oxide moieties, and from 0 to 1 glyceryl moiety, and $R_2$ and $R_3$ contain from 1 to 3 carbon atoms and from 0 to 1 hydroxy group, e.g., methyl, ethyl, propyl, hydroxyethyl, or hydroxypropyl radicals. The arrow in the formula is a conventional representation of a semipolar bond. Examples of amine oxides suitable for use in this invention include dimethyloctadecylamine oxide, oleyldi(methyl) amine oxide, dimethylhexadecylamine oxide, behenyldimethylamine oxide.

5. Long chain tertiary phosphine oxides corresponding to the following general formula:

$$RR'R''P \rightarrow O$$

wherein R contains an alkyl, alkenyl or monohydroxyalkyl radical ranging from 12 to 22 carbon atoms in chain length, from 0 to 10 ethylene oxide moieties and from 0 to 1 glyceryl moiety and R' and R'' are each alkyl or monohydroxyalkyl groups containing from about 1 to 3 carbon atoms. The arrow in the formula is a conventional representation of a semipolar bond.

6. Long chain dialkyl sulfoxides containing one short chain alkyl or hydroxy alkyl radical of from 1 to 3 carbon atoms (usually methyl) and one long hydrophobic chain which include alkyl, alkenyl, hydroxy alkyl, or keto alkyl radicals containing from 12 to 20 carbon atoms, from 0 to 10 ethylene oxide moieties and from 0 to 1 glyceryl moiety.

7. Silicone copolyols which may be polyalkylene oxide modified polydimethylsiloxanes of the following formulae:

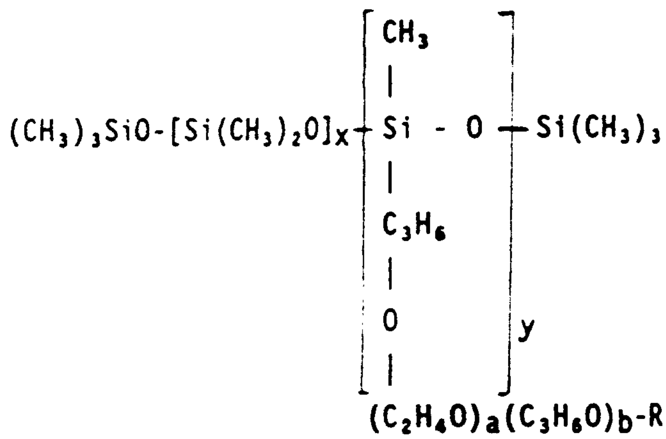

and

$R' - Si\{[-O\ Si(CH_3)_2]_x-(OC_2H_4)_a - (OC_3H_6)_b - OR'']_3$

wherein R is hydrogen, an alkyl group having from 1 to 12 carbon atoms, an alkoxy group having from 1 to 6 carbon atoms or a hydroxyl group; R' and R'' are alkyl groups having from 1 to 12 carbon atoms; x is an integer of from 1 to 100, preferably from 20 to 30; y is an integer of 1 to 20, preferably from 2 to 10; and a and b are integers of from 0 to 50, preferably from 20 to 30.

Dimethicone copolyols among those useful herein are disclosed in the following patent documents: U.S. Patent 4,122,029; U.S. Patent 4,265,878; and U.S. Patent 4,421,769. Such dimethicone copolyol materials are also disclosed, in hair compositions, in GB-A-2,066,659 and Canadian Patent 727,588. Commercially available dimethicone copolyols which can be used herein, include Silwet Surface Active Copolymers (manufactured by the Union Carbide Corporation); and Dow Corning Silicone Surfactants (manufactured by the Dow Corning Corporation).

8. Amide surfactants which include the ammonia, monoethanol, diethanol, and other alkanol amides of fatty acids having an acyl moiety of from 8 to 22 carbon atoms and represented by the general formula:

$R_1-CO-N(H)_{m-1}(R_2OH)_{3-m}$

wherein $R_1$ is a saturated or unsaturated, aliphatic hydrocarbon radical having from 7 to 21, preferably from 11 to 17 carbon atoms; $R_2$ represents a $C_{1-4}$ alkalene group; and m is 1, 2 or 3, preferably 1. Specific examples of said amides are mono-ethanol coconut fatty acids amide and diethanol dodecyl fatty acid amide. These acyl moieties may be derived from naturally occurring glycerides, e.g., coconut oil, palm oil, soybean oil and tallow, but can be derived synthetically, e.g., by the oxidation of petroleum, or by hydrogenation of carbon monoxide by the Fischer-Tropsch process. The monoethanol amides and diethanolamides of $C_{18-22}$ fatty acids are preferred.

Cationic surfactants useful in vehicle systems of the compositions of the present invention, contain amino or quaternary ammonium hydrophilic moieties which are positively charged when dissolved in the aqueous composition of the present invention. Cationic surfactants among those useful herein are disclosed in the following documents: M.C. Publishing Co., McCutcheon's, Detergents & Emulsifiers, (North American Edition 1979); Schwartz, et al., Surface Active Agents, Their Chemistry and Technology, New York: Interscience Publishers, 1949; U.S. Patent 3,155,591; U.S. Patent 3,929,678; U.S. Patent 3,959,461; and U.S. Patent 4,387,090.

Among the quaternary ammonium-containing cationic surfactant materials useful herein are water-insoluble surfactants of the general formula:

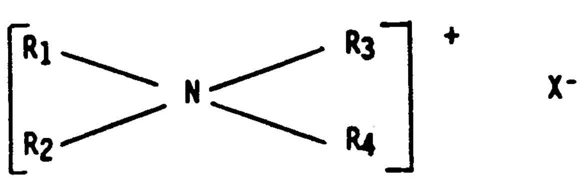

wherein $R_1$-$R_4$ can independently be selected from an aliphatic group of from 1 to 22 carbon atoms, $C_1$-$C_3$ alkyl, hydroxyalkyl, polyalkoxy or an aromatic, aryl or alkylaryl group having from 12 to 22 carbon atoms; and X is an anion selected from halogen, acetate, phosphate, nitrate and alkylsulfate radicals. The aliphatic groups may contain, in addition to carbon and hydrogen atoms, ether linkages, and other groups such as amino groups.

Other quaternary ammonium salts useful herein have the formula:

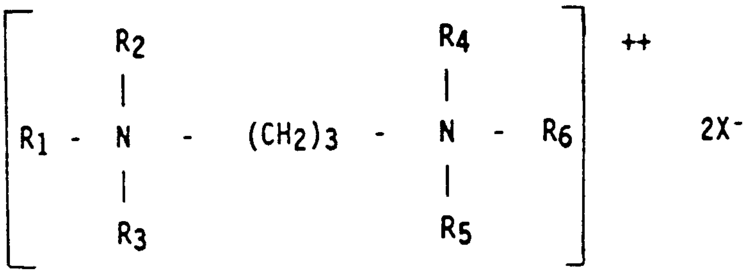

wherein $R_1$ is an aliphatic group having from 16 to 22 carbon atoms, $R_2$, $R_3$, $R_4$, $R_5$, and $R_6$ are selected from hydrogen and alkyl having from 1 to 4 carbon atoms, and X is an ion selected from halogen, acetate, phosphate, nitrate and alkyl sulfate radicals. Such quaternary ammonium salts include tallow propane diammonium dichloride.

Preferred quaternary ammonium salts include dialkyldimethyl-ammonium chlorides, wherein the alkyl groups have from 12 to 22 carbon atoms and are derived from long-chain fatty acids, such as hydrogenated tallow fatty acid (tallow fatty acids yield quaternary compounds wherein $R_1$ and $R_2$ have predominately from 16 to 18 carbon atoms). Examples of quaternary ammonium salts useful in the present invention include ditallowdimethyl ammonium chloride, ditallowdimethyl ammonium methyl sulfate, dihexadecyl dimethyl ammonium chloride, di(hydrogenated tallow) dimethyl ammonium chloride, dioctadecyl dimethyl ammonium chloride, dieicosyl dimethyl ammonium chloride, didocosyl dimethyl ammonium chloride, di(hydrogenated tallow) dimethyl ammonium acetate, dihexadecyl dimethyl ammonium chloride, dihexadecyl dimethyl ammonium acetate, ditallow dipropyl ammonium phosphate, ditallow dimethyl ammonium nitrate, di-(coconutalkyl) dimethyl ammonium chloride, and stearyl dimethyl benzyl ammonium chloride. Ditallow dimethyl ammonium chloride, dicetyl dimethyl ammonium chloride, stearyl dimethyl benzyl ammonium chloride and behenyl trimethyl ammonium chloride are preferred quaternary ammonium salts useful herein. Di-(hydrogenated tallow) dimethyl ammonium chloride is a particularly preferred quaternary ammonium salt for use in the present invention.

Salts of primary, secondary and tertiary fatty amines are also preferred cationic surfactant materials for use herein. The alkyl groups of such amines preferably have from 12 to 22 carbon atoms, and may be substituted or unsubstituted. Secondary and tertiary amines are preferred, tertiary amines are particularly preferred. Such amines, useful herein, include stearamido propyl dimethyl amine, diethyl amino ethyl stearamide, dimethyl stearamine, dimethyl soyamine, soyamine, tridecyl amine, ethyl stearylamine, ethoxylated (2 moles E.O.) stearylamine, dihydroxyethyl stearylamine, and arachidylbehenylamine. Suitable amine salts include the halogen, acetate, phosphate, nitrate, citrate, lactate and alkyl sulfate salts. Such salts include stearylamine hydrochloride, soyamine chloride, stearylamine formate, N-tallowpropane diamine dichloride and stearamidopropyl dimethylamine citrate. Cationic amine surfactants included among those useful in the present invention are disclosed in U.S. Patent 4,275,055.

Zwitterionic surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents contains from 8 to 18 carbon atoms and one contains in anionic water-solubilizing group, e.g., carboxy, sulfonate, sulfate, phosphate, or phosphonate. A general formula for these compounds is:

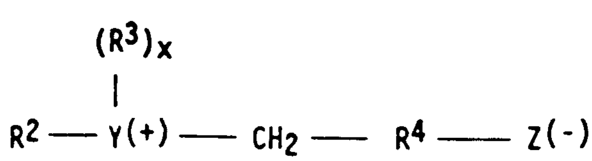

wherein $R^2$ contains an alkyl, alkenyl, or hydroxy alkyl radical of from 8 to 18 carbon atoms, from 0 to 10 ethylene oxide moieties and from 0 to 1 glyceryl moiety; Y is selected from nitrogen, phosphorus, and sulfur atoms; $R^3$ is an alkyl or monohydroxyalkyl group containing about 1 to 3 carbon atoms; X is 1 when Y is a sulfur atom, and 2 when Y is a nitrogen or phosphorus atom; $R^4$ is an alkylene or hydroxyalkylene of from about 1 to about 4 carbon atoms and Z is a radical selected from carboxylate, sulfonate, sulfate, phosphonate, and phosphate groups.

Other zwitterionics such as betaines are also useful in the present invention. Examples of betaines useful herein include the high alkyl betaines, such as stearyl dimethyl carboxymethyl betaine, behenyl dimethyl carboxymethyl betaine, stearyl bis-(2-hydroxypropyl) carboxymethyl betaine, oleyl dimethyl gamma-carboxypropyl betaine. The sulfobetaines may be represented by behenyl dimethyl sulfopropyl betaine, stearyl dimethyl sulfopropyl betaine, and the like; hydrogenated tallow dimethyl betaine; amidobetaines and amidosulfobetaines, wherein the $RCONH(CH_2)_3$ radical is attached to the nitrogen atom of the betaine are also useful in this invention.

Examples of amphoteric surfactants which can be used in the vehicle systems of the compositions of the present invention are those which are broadly described as derivatives of aliphatic secondary and tertiary amines in which the aliphatic radical can be straight or branched chain and wherein one of the aliphatic substituents contains from 8 to 18 carbon atoms and one contains an anionic water solubilizing group, e.g., carboxy, sulfonate, sulfate, phosphate, or phosphonate.

Examples of preferred water-insoluble surfactants for use in the present invention are stearamide DEA, cocamide MEA, dimethyl stearamine oxide, glyceryl monooleate, sucrose stearate, PEG-2 stearamine, Ceteth-2, a polyethylene glycol ether of cetyl alcohol of the formula $CH_3\text{-}(CH_2)_{14}\text{-}CH_2\text{-}(OCH_2CH_2)_n\text{-}OH$, where n has an average value of 2 (commerically available under the trade name Brij 52 from ICI Americas), glycerol stearate citrate, dihydrogenated tallow dimethyl ammonium chloride, Poloxamer 181, a polyoxyethylene, polyoxypropylene block polymer of the formula

$$HO - (CH_2 - CH_2 - O)_x\ (\underset{\displaystyle CH_3}{CH} - CH_2 - O)_y\ (CH_2 - CH_2O)_zH;$$

wherein on average x = 3, y = 30 and z = 3 (commercially available from BASF Wyandotte under the trade name Pluronic L-61), hydrogenated tallow dimethyl betaine, and hydrogenated tallow amide DEA.

The water-insoluble surfactant is used with the primary thickener of the present invention at from 0.02% to 10.0%, preferably from 0.05% to 3.0%, most preferably from 0.05% to 2.0%, of the composition.

Solvent

A third essential component in the vehicle systems of the present invention is a solvent which is compatible with the other components in the present compositions. Generally the solvent will comprise water or a water-lower alkanol mixture. The solvent is present in the compositions of the present invention at a level of from 65% to 99% by weight of the cosmetic composition.

The other vehicle components are dispersed or mixed in the solvent to provide an optimum thick rheology to cosmetic compositions formulated therewith which mimics the gel-network rheology of typical hair conditioning compositions. This rheology is characterized by a shear stress of from 0 to 50 pascal, over a shear rate range of 0.04 $sec^{-1}$ to 25 $sec^{-1}$. The rheology is measured using a Bohlin Rheometer VOR with the following cone and plate set-up: cone has a 2.5 degree angle, plate is 30mm in diameter, the gap between the truncated cone and plate is set at 70µm, and the torque bar used is 20.148 g-cm. The sample amount is 0.35ml and the sample is syringed onto the center of the plate. The system used is as follows: there is no initial delay time, the strain delay time is 25 sec, the integration time is 5 sec, the sensitivity is set at 1X, the shear sweep is up, the shear range is from about 0.0405 $sec^{-1}$ to 25.53 $sec^{-1}$ (shear No. = 11 to 39), and the temperature is maintained constant between series at ambient temperature (20°C to 25°C).

Additional Thickener

The present vehicle systems can also comprise an additional thickening component, which comprises a water-soluble polymeric material, having a molecular weight greater than 20,000. By "water-soluble polymer" is meant that the material will form substantially a clear solution in water at a 1% concentration at

25°C and the material will increase the viscosity of the water. Examples of water-soluble polymers which may desirably be used as an additional thickening component in the present vehicle systems, are hydroxyethylcellulose, hydroxypropyl cellulose, hydroxypropyl methylcellulose, polyethylene glycol, polyacrylamide, polyacrylic acid, polyvinyl alcohol, polyvinyl pyrrolidone K-120, dextrans, for example Dextran purified crude Grade 2P, available from D&O Chemicals, carboxymethyl cellulose, plant exudates such as acacia, ghatti, and tragacanth, seaweed extracts such as sodium alginate, propylene glycol alginate and sodium carrageenan, and Ucare JR-polymer (a cationic modified hydroxyethyl cellulose available from Union Carbide). Preferred as the optional additional thickener for the present vehicle systems are natural polysaccharide materials. Examples of such materials are guar gum, locust bean gum, and xanthan gum. Also preferred as the additional thickener in the present compositions is hydroxyethyl cellulose having a molecular weight of about 700,000. It is important that these polymer materials not contain cellulase as this may interfere with obtaining optimum viscosities.

The additional thickening component, if present in the cosmetic compositions of the present invention, is at a level of from 0.3% to 5.0%, preferably from 0.4% to 3.0%.

It is important that these additional polymer materials be well-hydrated and dispersed in the present compositions.

Rheological Aid

The vehicle systems of the present invention preferably also contain a material which provides additional rheological benefits to the cosmetic compositions formulated therewith. These materials are chelating agents. In general, such materials include monodentate and multidentate agents. Specific examples of useful chelating agents include ethylenediaminetetraacetic acid (EDTA), and salts thereof, nitrilotriacetic acid (NTA) and salts thereof, hydroxyethyl ethylene diamine triacetic acid (HEEDTA) and salts thereof, diethylene triamine pentaacetic acid (DTPA) and salts thereof, diethanol glycine (DEG) and salts thereof, ethanoldiglycine (EDG) and salts thereof, citric acid and salts thereof, phosphoric acid and salts. The most preferred of these is EDTA. The chelating agents tend to make the vehicle systems of the present invention smoother and less gelatinous in consistency.

If a chelating agent is present as a rheological aid in the compositions of the present invention it is present at a level of from 0.05% to 1.0%, preferably from 0.05% to 0.3%, of the composition.

Distributing Aid

An additional component in the vehicle systems of the present invention is a material which acts as a distributing aid for the composition. Such a material helps to distribute the cosmetic composition onto the hair or skin avoiding localized deposition of the active component onto the hair or skin. Without such a component in a composition, some active components in the composition would not be deposited and spread out as evenly, and hence, would not be quite as effective.

Distributing aid materials useful in the present invention are actually a subclass of the class of materials used as the optional water-soluble polymer additional thickener in the present invention. This subclass is defined as follows: water-soluble polymer materials having high molecular weight, i.e., greater than 1,000,000; and/or strong ionic character. By strong ionic character is meant that the material conducts electricity at greater than 30 millivolts. This can be measured by evaluating conductance of a 1% solution of polymer in DRO (double reverse osmosis) water preserved with 0.03% Kathon CG (a preservative available from Rohm & Haas) using a calibrated Corning 130 pH meter. The probes used were as follows: the reference electrode is an Orion Model 9001 single junction. The pH electrode is an Orion Model 9161, silver-silver chloride. The probes are set 3/8 of an inch apart. The pH meter is set to millivolt readings. The absolute measurement is recorded after 4 minutes immersion.

Examples of water soluble polymer materials which meet these requirements and hence, can act as distributing aids in the present compositions include xanthan gum; Dextran purified crude Grade 2P available from D&O chemicals; carboxymethyl celluloses; for example, CMC's 4H1F, 4M6f, 7HF, 7M8SF, 7LF, 9H4F, 9M8, 12M8P, 16M31, (all available from Aqualon); plant exudates such as acacia, ghatti and tragacanth; seaweed extracts such as sodium alginate, propylene glycol alginate, and sodium carrageenan; high molecular weight hydroxyethyl celluloses such as Natrosol 250H and Natrosol 250HHR (available from Aqualon); and pectin.

Because the class of materials which may act as distributing aids in the present invention is a subset of the optional water-soluble additional thickener, the materials in this subclass may be used to provide both benefits to the composition. For example, xanthan gum is a water-soluble natural polysaccharide material

which additionally has a high molecular weight. Hence, this material could be used by itself to provide both additional thickening benefits and distributing benefits. However, it may be necessary to use such materials at slightly higher levels to provide both benefits.

It is also possible to use two separate materials as the optional water-soluble polymer additional thickener and the distributing aid of the present invention. This would be done when the water-soluble polymer additional thickener was not a high molecular weight material or of strong ionic character. Locust bean gum is such a material. A distributing aid such as xanthan gum could be used with locust bean gum to provide the additional distributing benefits.

If a distributing aid is present in the cosmetic compositions of the present invention, it should be present at a level of from 0.02% to 2.5%, preferably from 0.05% to 1.0%, of the cosmetic composition. If the distributing aid is bifunctional, i.e., acting as both the optional additional thickener and the distributing aid it should be present at a level of from 0.2% to 5.0% of the composition.

A distributing aid is particularly useful in hair care compositions of the present invention especially rinse-off hair conditioners. The distributing aid helps to spread some hair conditioning components evenly over the hair.

The present vehicle systems and cosmetic compositions formulated therewith must be substantially free of water-soluble surfactants. These materials are not compatible with the vehicle systems of the present composition. By "substantially free of water-soluble surfactants" is meant that the compositions comprise less than an amount of such surfactants that will destroy the present unique desirable rheology that is the object of the prevent invention. Generally, this will mean that the present compositions comprise no more than 1%, preferably no more than 0.5%, of such materials. Examples of specific water-soluble surfactant materials that can be particularly harmful to the present vehicle systems are alkyl sulfates and ethoxylated alkyl sulfates, such as ammonium lauryl sulfate; amphoteric surfactants which are derivatives of aliphatic secondary and tertiary amines; nonionic surfactants produced by the condensation of alkylene oxide groups with an organic hydrophilic compound, such as laureth-23 (sold under the trademark Brij 35 by ICI Americas); and high alkyl betaines, sulfo betaines, amido betaines and amido sulfobetaines, such as cetyl betaine. Such materials are commonly used in hair shampoo compositions.

The present vehicle systems and cosmetic compositions formulated therewith are also preferably substantially free of fatty alcohol materials, such as stearyl-, cetyl-, myristyl-, behenyl-, lauryl-, and oleyl alcohol. By "substantially free of fatty alcohol materials" is meant that the compositions of the present invention comprise no more than 1% of these materials. These materials are commonly used in vehicle systems for hair conditioner products. However, these materials are undesirable because they tend to deposit on the hair and leave the hair feeling dirty after use. These materials are not required and are not desirable in the present vehicle systems, as they are thickened with alternative materials which do not deposit on hair.

The present vehicle systems can be used in essentially any cosmetic products having a thick gel-network type rheology and which are used to deliver some active component onto the hair or skin. Such compositions would include skin moisturizing lotions, sunscreen compositions, and skin cleansing compositions. However, cosmetic compositions most desirably used with the present vehicle systems are hair care products, especially rinse-off hair care products where some active hair care component is to be deposited onto the hair but the vehicle carrying that component is desirably rinsed off of the hair with little or no deposition of the vehicle material onto the hair.

Generally, the present vehicle systems will not be useful in typical shampoo compositions since these compositions contain high levels of water-soluble surfactants, which as discussed ***supra***, are incompatible with the present vehicle systems. However, the present vehicle systems are useful in typical hair coloring compositions, hair tonic or gel compositions, hair mousse compositions, and especially hair conditioning compositions.

Active Cosmetic Component

The cosmetic compositions of the present invention generally will comprise some active component which provides some benefit to the hair or skin. Such materials may include moisturizing agents, sunscreen agents, cleaning agents (that are compatible with the present vehicle systems), and especially hair conditioning agents, hair styling agents, antidandruff agents, hair growth promoters, hair dyes and pigments, or perfumes.

A wide variety of conventional sunscreening agents are suitable for use in the cosmetic compositions of the present invention. Segarin, et al., at Chapter VIII, pages 189 et seq., of Cosmetics Science and Technology, disclose numerous suitable agents. Specific suitable sunscreening agents include, for example:

p-aminobenzoic acid, its salts and its derivatives; anthranilates; salicylates; cinnamic acid derivatives; dihydroxycinnamic acid derivatives: trihydroxycinnamic acid derivatives: hydrocarbons; dibenzalacetone and benzalacetophenone; naphtholsulfonates; dihydroxy-naphtholic acid and its salts; coumarin derivatives; diazoles; quinine salts; quinoline derivatives; hydroxy- or methoxy-substituted benzophenones; uric and vilouric acids; tannic acid and its derivatives; hydroquinone; and benzophenones.

Of these, 2-ethylhexyl p-methoxycinnamate, 4,4'-t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone,ethyl-4-[bis(hydroxypropyl)]-aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethyl-aminobenzoic acid or aminobenzoate, 2-ethylhexyl p-dimethylaminobenzoate, 2-phenylbenzimidazole-5-sulfonic acid 2-(p-dimethyl-aminophenyl)-5-sulfonicbenzoxazoic acid, and mixtures of these compounds are particularly useful.

Examples of antidandruff aids suitable for use with the vehicle systems of the present invention include zinc pyrithione, sulphur, and selenium sulfide. One example of a hair growth promoter suitable for use with the vehicle systems of the present invention is Minoxidil, (6-amino-1, 2-dihydro -1-hydroxy-2-imino-4-piperidino pyrimide) available from Upjohn. Hair oxidizing (bleaching) agents, such as hydrogen peroxide, perborate and persulfate salts, and hair reducing agents such as thioglycolates may also be used.

Examples of hair conditioning materials suitable for use in the vehicle systems of the present invention are volatile liquid hydrocarbon or silicone agents.

These materials preferably have a boiling point in the range of 99°C to 260°C and have a solubility in water of less than 0.1%. The hydrocarbons may be either straight or branched chain and may contain from 10 to 16, preferably from 12 to 16 carbon atoms. Examples of suitable hydrocarbons are decane, dodecane, tetradecane, tridecane and mixtures thereof.

The volatile silicones useful as the active hair treating component in the compositions of the present invention may be either a cyclic or a linear polydimethylsiloxane. The number of silicon atoms in the cyclic silicones is preferably from 3 to 7, more preferably 4 or 5.

The general formula for such silicones is

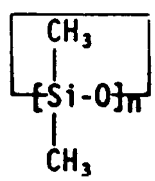

wherein n = 3-7. The linear polydimethylsiloxanes have from 3 to 9 silicon atoms and have the general formula:

$(CH_3)_3Si\text{-}O\text{-}[\text{-}Si(CH_3)_2\text{-}O\text{-}]_n\text{-}Si(CH_3)_3$ n = 1-7.

Silicones of the above type, both cyclic and linear, are available from Dow Corning Corporation, Dow Corning 344, 345 and 200 fluids; Union Carbide, Silicone 7202 and Silicone 7158; and Stauffer Chemical, SWS-03314.

The linear volatile silicones generally have viscosities of less than 5 centipoise at 25°C while the cyclic materials have viscosities less than 10 centipoise. "Volatile" means that the material has a measurable vapor pressure. A description of volatile silicones is found in Todd and Byers, "Volatile Silicone Fluids for Cosmetics", *Cosmetics and Toiletries*, Vol. 91, January 1976, pp. 27-32.

The volatile agent may be present in the compositions of this invention at a level of from 1% to 20%, preferably from 2% to 15%. The volatile silicones are the preferred volatile agents.

Nonvolatile silicone fluids are also useful as the active hair care component in the compositions of the present invention. Examples of such materials include polydimethylsiloxane gums, aminosilicones and phenylsilicones. More specifically, materials such as polyalkyl or polyaryl siloxanes with the following structure:

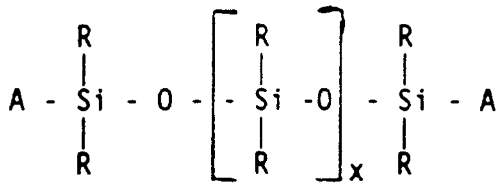

wherein R is alkyl or aryl, and x is an integer from 7 to 8,000 may be used. A represents groups which block the ends of the silicone chains.

The alkyl or aryl groups substituted on the siloxane chain (R) or at the ends of the siloxane chains (A) may have any structure as long as the resulting silicones remain fluid at room temperature, are hydrophobic, are neither irritating, toxic nor otherwise harmful when applied to the hair, are compatible with the other components of the composition, are chemically stable under normal use and storage conditions, and are capable of being deposited on and of conditioning hair.

Suitable A groups include methyl, methoxy, ethoxy, propoxy, and aryloxy. The two R groups on the silicone atom may represent the same group or different groups. Preferably, the two R groups represent the same group. Suitable R groups include methyl, ethyl, propyl, phenyl, methylphenyl and phenylmethyl. The preferred silicones are polydimethyl siloxane, polydiethylsiloxane, and polymethylphenylsiloxane. Polydimethylsiloxane is especially preferred.

Suitable methods for preparing these silicone materials are disclosed in U.S. Patents 2,826,551 and 3,964,500 and references cited therein. Silicones useful in the present invention are also commercially available. Suitable examples include Viscasil, a trademark of the General Electric Company and silicones offered by Dow Corning Corporation and by SWS Silicones, a division of Stauffer Chemical Company.

Other useful silicone materials include materials of the formula:

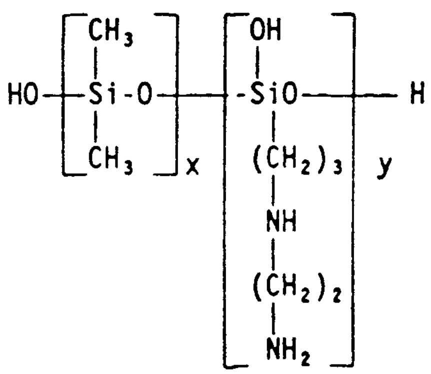

(I)

in which x and y are integers which depend on the molecular weight, the average molecular weight being approximately between 5,000 and 10,000. This polymer is also known as "amodimethicone".

Other silicone cationic polymers which can be used in the present composition correspond to the formula:

$(R_1)_aG_{3-a}\text{-Si-(-OSiG}_2)_n\text{-(OSiG}_b(R_1)_{2-b})_m\text{-O-SiG}_{3-a}(R_1)_a$

in which G is chosen from hydrogen, phenyl, OH, $C_1$-$C_8$ alkyl and preferably methyl; a denotes 0 or an integer from 1 to 3, and preferably equals 0;

b denotes 0 or 1 and preferably equals 1; the sum n + m is a number from 1 to 2,000 and preferably from 50 to 150, n being able to denote a number from 0 to 1,999 and preferably from 49 to 149 and m being able to denote an integer from 1 to 2,000 and preferably from 1 to 10;

$R_1$ is a monovalent radical of formula $C_qH_{2q}L$ in which q is an integer from 2 to 8 and L is chosen from the groups

$-N(R_2)CH_2-CH_2-N(R_2)_2$
$-N(R_2)_2$
$-\overset{+}{N}(R_2)_3A^-$

$-\overset{+}{N}(R_2)CH_2-CH_2-\overset{+}{N}R_2H_2A^-$

in which $R_2$ is chosen from hydrogen, phenyl, benzyl, a saturated hydrocarbon radical, preferably an alkyl radical containing from 1 to 20 carbon atoms, and $A^-$ denotes a halide ion.

These compounds are described in greater detail in EP-A-95,238. An especially preferred polymer corresponding to this formula is the polymer known as "trimethylsilylamodimethicone" of formula:

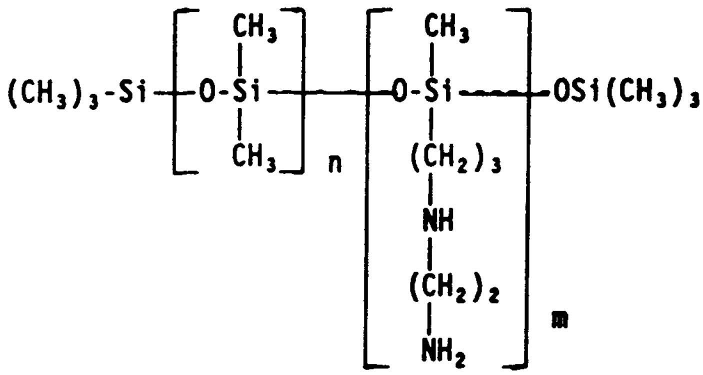

(II)

Compositions of the present invention may comprise up to 1.0% of a trimethylsilyl amodimethicone silicone conditioning material.

Other silicone cationic polymers which can be used in the present compositions correspond to the formula:

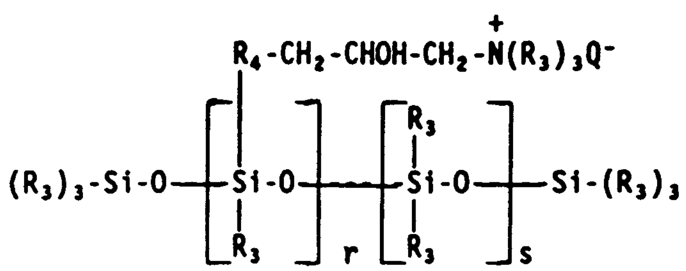

(III)

in which $R_3$ denotes a monovalent hydrocarbon radical having from 1 to 18 carbon atoms, and more especially an alkyl or alkenyl radical such as methyl;

$R_4$ denotes a hydrocarbon radical such as, preferably a $C_1$-$C_{18}$ alkylene radical or a $C_1$-$C_{18}$, and preferably $C_1$-$C_8$, alkyleneoxy radical;

$Q^-$ is a halide ion, preferably chloride;

r denotes an average statistical value from 2 to 20, preferably from 2 to 8;

s denotes an average statistical value from 20 to 200, and preferably from 20 to 50.

These compounds are described in greater detail in U.S. Patent 4,185,017.

A polymer of this class which is especially preferred is that sold by UNION CARBIDE under the name "UCAR SILICONE ALE 56".

Silicone conditioning agents are used in the present compositions at levels of from 0.1% to 18%, preferably from

0.5% to 15%. Preferred silicone conditioning agents for use in the present compositions comprise combinations of volatile silicone fluids having viscosities of less than 10 centipoise, and from 0.015% to 9.0%, preferably from 0.5% to 2.0%, of silicone gums having viscosities of greater than 1,000,000 centipoise, at ratios of volatile fluid to gum of from 90:10 to 10:90, preferably from 85:15 to 50:50.

Alternative preferable nonvolatile silicone materials for use in the present invention comprise non-volatile silicone fluids having viscosities of less than 100,000 cP (centipoise), and from 0.015% to 9.0%, preferably from 0.5% to 2.0%, of silicone gums having viscosities greater than 1,000,000 cP, especially polydimethylsiloxane gums and polyphenylmethylsiloxane gums, at ratios of non-volatile fluid to gum of from 70:30 to 30:70, preferably from 60:40 to 40:60.

Other preferred active hair care materials for use with the vehicle systems of the present invention are silicone polymer materials which provide both style retention and conditioning benefits to the hair. Although silicone fluids are useful in the present compositions, preferred silicone polymers are rigid silicone polymers. Such materials are described in U.S. Patent 4,902,499 and U.S. Patent 4,906,459.

Some examples of such materials include, but are not limited to, filler reinforced polydimethyl siloxane gums including those having end groups such as hydroxyl; cross linked siloxanes, such as organic substituted silicone elastomers; organic substituted siloxane gums, including those having end groups such as hydroxyl; resin reinforced siloxanes; and cross linked siloxane polymers.

The rigid silicone polymers useful in the present invention have complex viscosities of at least $2 \times 10^5$ poise (P), preferably about $1 \times 10^7$ poise, where complex viscosity is measured by subjecting a sample to oscillatory shear at a fixed frequency of 0.1 rad/sec at 25°C using a Rheometric Fluids Spectrometer® measuring films having a thickness of about 1 millimeter. The resulting viscous and elastic force responses are combined to determine the complex modulus which is divided by the imposed frequency to compute the complex viscosity.

A preferred siloxane gum useful in the present invention is a diphenyl-dimethyl polysiloxane gum having a molecular weight of at least 500,000, and must be diphenyl substituted to the extent of 3% or more, preferably at least 5%.

The siloxane gums may also be filler reinforced to provide additional rigidity. Silica is the preferred filler. Generally such reinforced gums comprise up to about 15-20% silica.

Silicone elastomers useful in the compositions of the present invention are the materials described in U.S. Patent 4,221,688. The actual material described in the patent and what can be put into the present compositions is an aqueous emulsion which dries to form an elastomer upon removal of the water.

The silicone emulsion has a continuous water phase in which there is a dispersed phase which comprises an anionically stabilized hydroxylated polyorganosiloxane, a colloidal silica and a catalyst. The pH of the emulsion should be in the range of from 9 to 11.5, preferably from 10.5 to 11.2. The solids content of the emulsion is generally from 20% to 60%, preferably from 30% to 50%. The amount of colloidal silica present for each 100 parts by weight of the polydiorganosiloxane is from 1 to 150 parts. On the same basis the amount of a diorganotindicarboxylate (e.g., dioctyl tindilaurate) catalyst is from 0.1 to 2 parts. The elastomer emulsion is used in an amount of from 0.1% to 5%, preferably from 0.5% to 4%, of the total composition.

Silicone resins useful in the present compositions are silicone polymers with a high degree of crosslinking introduced through the use of trifunctional and tetrafunctional silanes. Typical silanes used in the manufacture of resins are monomethyl, dimethyl, monophenyl, diphenyl, methylphenyl, monovinyl, and methylvinyl chlorosilanes, together with tetrachlorosilane. A preferred resin is one offered by General Electric as GE SR545. This resin is provided as a solution in toluene which is stripped prior to the resin's use.

Other rigid silicone polymers of use herein are those siloxanes which have been sparingly crosslinked but are still soluble in solvents such as cyclomethicone. Precursors for the rigid material can be any high molecular weight polydimethyl siloxanes, polydimethyl siloxanes containing vinyl groups and other siloxanes. Methods of crosslinking include heat curing with organic peroxides such as dibenzoyl peroxide and di-t-butyl peroxide, heat vulcanization with sulfur, and high-energy radiation.

Generally, the silicone gum, if used in the present compositions, is dissolved in a volatile carrier, or mixtures thereof, prior to incorporation into the hair care compositions. Preferably, the volatile carrier is present in the hair care composition at from 0.1% to 20% of the hair care composition. These materials can comprise the volatile liquid hydrocarbon or silicone fluids described *supra*.

Preferably the rigid silicone polymer and carrier comprises from 0.1% to 2.5% of a polydimethylsiloxane gum; from 0.02% to 0.7% of fumed silica, and from 0.4% to 18% of a volatile silicone carrier.

Alternative hair conditioning materials may be used in the present compositions. Such materials include cationic surfactant conditioning agents. These materials are actually a subclass of the water-insoluble surfactant component of the present compositions as described *supra*. Preferred cationic surfactants for use as hair conditioning agents in the present compositions are quaternary ammonium-containing cationic surfactant materials. These materials are described in more detail *supra*. If such a material is included in the present compositions it will be present at levels up to 2.5%, preferably at from 0.5% to 2.0%, by weight of the composition. The preferred quaternary ammonium-containing cationic surfactant for use herein is di-(hydrogenated) tallow dimethyl ammonium chloride. When these materials are included in the compositions of the present invention, they are included as a part of the water-insoluble surfactant component, and provide hair conditioning, as well as vehicle, benefits.

Alternative cationic water-insoluble surfactant hair conditioning agents that may be used in the present compositions are salts of primary, secondary, and tertiary fatty amines. These materials are described in more detail *supra*. The preferred of these materials is stearamido propyl dimethyl amine. A commercially available material is sold under the trade name Lexamine® by Inolex Company. Preferably, up to 1% of such materials may be used in the present compositions to provide conditioning benefits.

Hydrolyzed animal protein hair conditioning agents may also be included in the present compositions. Such materials are present in the compositions at levels of from 0.1% to 1.5%. An example of a commercially available material is sold under the tradename Crotein Q® from Croda, Inc.

Fatty alcohols are known hair conditioning agents and may be included in the present compositions. However, as described *supra* such materials tend to deposit on hair and leave hair feeling dirty after use. Hence, fatty alcohol materials are not included in the compositions of the present invention at levels greater than 1%.

Combinations of the aforementioned conditioning agents may also be used in the present compositions.

Highly preferred active hair care materials for use with the vehicle systems of the present invention are hair holding/styling polymers. Such polymers should have a weight average molecular weight of from 10,000 to 1,000,000 and preferably, have a Tg of at least -20 °C. As used herein, the abbreviation "Tg" refers to the glass transition temperature of the non-silicone backbone, and the abbreviation "Tm" refers to the crystalline melting point of the non-silicone backbone, if such a transition exists for a given polymer.

Preferred polymers comprise a vinyl polymeric backbone having a Tg or a Tm above -20 °C and, grafted to the backbone, a polydimethylsiloxane macromer having a weight average molecular weight of from 1,000 to 50,000, preferably from 5,000 to 40,000, most preferably from 10,000 to 20,000. The polymer is such that when it is formulated into the finished hair care composition, when dried, the polymer phase separates into a discontinuous phase which includes the polydimethylsiloxane macromer and a continuous phase which includes the backbone. It is believed that this phase separation property provides a specific orientation of the polymer on hair which results in the desired hair conditioning and setting benefits.

In its broadest aspect, the copolymers utilized in the present application comprise C monomers together with monomers selected from A monomers, B monomers, and mixtures thereof. These copolymers contain at least A or B monomers together with C monomers, and preferred copolymers contain A, B and C monomers.

Examples of useful copolymers and how they are made are described in detail in U.S. Patent 4,693,935 and U.S. Patent 4,728,571. These copolymers are comprised of monomers A, C and, optionally, B, which are defined as follows. A, when used, is at least one free radically polymerizable vinyl monomer or monomers. B, when used, comprises at least one reinforcing monomer copolymerizable with A and is selected from polar monomers and macromers having a Tg or a Tm above -20 °C. When used, B may be up to 98%, preferably up to 80%, more preferably up to 20%, of the total monomers in the copolymer. Monomer C comprises from 0.01% to 50.0% of the total monomers in the copolymer.

Representative examples of A monomers are acyrlic or methacrylic acid esters of $C_1$-$C_{18}$ alcohols, such as methanol, ethanol, 1-propanol, 2-propanol, 1-butanol, 2-methyl-1-propanol, 1-pentanol, 2-pentanol, 3-pentanol, 2-methyl-1-butanol, 1-methyl-1-butanol, 3-methyl-1-butanol, 1-methyl-1-pentanol, 2-methyl-1-pentanol, 3-methyl-1-pentanol, t-butanol, cyclohexanol, 2-ethyl-1-butanol, 3-heptanol, benzyl alcohol, 2-octanol, 6-methyl-1-heptanol, 2-ethyl-1-hexanol, 3,5-dimethyl-1-hexanol, 3,5,5-trimethyl-1-hexanol, 1-decanol, 1-dodecanol, 1-hexadecanol, 1-octadecanol, the alcohols having from 1-18 carbon atoms with the average number of carbon atoms being from 4-12; styrene; vinyl acetate; vinyl chloride; vinylidene chloride; acrylonitrile; alpha-methylstyrene; t-butylstyrene; butadiene; cyclohexadiene; ethylene; propylene; vinyl toluene; and mixtures thereof. Preferred A monomers include n-butyl methacrylate, isobutyl methacrylate, 2-ethylhexyl methacrylate, methyl methacrylate, t-butylacrylate, t-butylmethacrylate, and mixtures thereof.

Representative examples of B monomers include acrylic acid, methacrylic acid, N,N-dimethylacrylamide, dimethylaminoethyl methacrylate, quaternized dimethylaminoethyl methacrylate, methacrylonitrile, polystyrene macromer, methacrylamide, maleic anhydride and its half esters, itaconic acid, acrylamide, acrylate alcohols, hydroxyethyl methacrylate, diallyldimethyl ammonium chloride, vinyl pyrrolidone, vinyl ethers (such as methyl vinyl ether), maleimides, acylactones, 2-ethyl-2-oxazoline, vinyl pyridine, vinyl imidazole, other polar vinyl heterocyclics, styrene sulfonate, and mixtures thereof. Preferred B monomers include acrylic acid, N,N-dimethylacrylamide, dimethylaminoethyl methacrylate, quaternized dimethylaminoethyl methacrylate, vinyl pyrrolidone, and mixtures thereof.

The C monomer has the general formula:

$$X(Y)_nSi(R)_{3-m}Z_m$$

wherein X is a vinyl group copolymerizable with the A and B monomers; Y is a divalent linking group; R is a hydrogen, lower alkyl, aryl or alkoxy; Z is a monovalent siloxane polymeric moiety having a number average molecular weight of at least 500, is essentially unreactive under copolymerization conditions and is pendant from the vinyl polymeric backbone, described above; n is 0 or 1; and m is an integer from 1 to 3. C has a weight average molecular weight of from 1,000 to 50,000, preferably from 5,000 to 40,000, most preferably from 10,000 to 20,000. Preferably, the C monomer has a formula selected from:

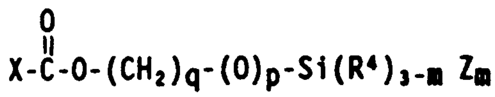

(a preferred monomer, particularly preferred when p = 0 and q = 3)

$X\text{-}Si(R^4)_{3-m}\ Z_m$

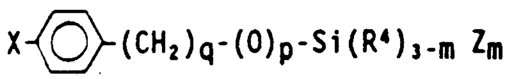

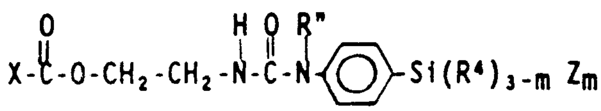

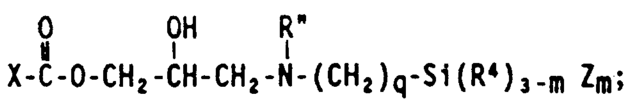

; and

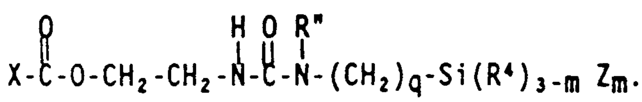

In those structures, m is 1, 2 or 3 (preferably m = 1); p is 0 or 1; R'' is alkyl or hydrogen; q is an integer from 2 to 6; s is an integer from 0 to 2; X is

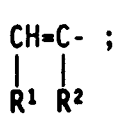

$R^1$ is hydrogen or -COOH (preferably $R^1$ is hydrogen); $R^2$ is hydrogen, methyl or $-CH_2COOH$ (preferably $R^2$ is methyl); Z is

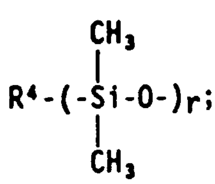

$R^4$ is alkyl, alkoxy, alkylamino, aryl, or hydroxyl (preferably $R^4$ is alkyl); and r is an integer from 5 to 700 (preferably r is about 250).

The preferred polymers useful in the present invention generally comprise from 0% to 98% (preferably from 5% to 98%, more preferably from 50% to 90%) of monomer A, from 0% to 98% (preferably from 7.5% to 80%) of monomer B, and from 0.1% to 50% (preferably from 0.5% to 40%, most preferably from 2% to 25%) of monomer C. The combination of the A and B monomers preferably comprises from 50.0% to 99.9% (more preferably 60% to 99%, most preferably from 75% to 95%) of the polymer. The composition of any particular copolymer will help determine its formulational properties. For example, polymers which are soluble in an aqueous formulation preferably have the composition: from 0% to 70% (preferably from 5% to 70%) monomer A, from 30% to 98% (preferably from 3% to 80%) monomer B, and from 1% to 40% monomer C. Polymers which are dispersible have the preferred composition: from 0% to 70% (more preferably from 5% to 70%) monomer A, from 20% to 80% (more preferably from 20% to 60%) monomer B, and from 1% to 40% monomer C.

Particularly preferred polymers for use in the present invention include the following (the weight percents below refer to the amount of reactants added in the polymerization reaction, not necessarily the amount in the finished polymer):

acrylic acid/n-butylmethacrylate/polydimethylsiloxane (PDMS) macromer-20,000 molecular weight (10/70/20 w/w/w) (I)

N,N-dimethylacrylamide/isobutyl methacrylate/PDMS macromer - 20,000 molecular weight (20/60/20 w/w/w) (II)

dlmethylaminoethyl methacrylate/isobutyl methacrylate/2-ethylhexyl-methacrylate/PDMS macromer-20,000 molecular weight (25/40/15/20 w/w/w/w) (III)

dimethylacrylamide/PDSM macromer-20,000 molecular weight (80/20 w/w) (IV)

t-butylacrylate/t-butylmethacrylate/PDMS macromer-10,000 molecular weight (56/24/20 w/w/w) (V)

t-butylacrylate/PDMS macromer-10,000 molecular weight (80/20 w/w) (VI)

t-butylacrylate/N,N - dimethylacrylamide/PDMS macromer-10,000 molecular weight (70/10/20 w/w/w)-(VII)

t-butylacrylate/acrylic acid/PDMS macromer-10,000 molecular weight (75/5/20 w/w/w) (VIII).

The particle size of the copolymer material of the present compositions may have some effect on performance in product. This, of course, will vary from copolymer to copolymer and from product to product.

The copolymers are preferably combined with a solvent for the copolymer prior to combination with the vehicle systems of the present invention.

The solvent selected must be able to dissolve or disperse the particular silicone copolymer being used. The nature and proportion of B monomer in the copolymer largely determines its polarity and solubility characteristics. The silicone copolymers can be designed, by appropriate combination of monomers, for formulation with a wide range of solvents. Suitable solvents for use in the present invention include, but are not limited to, water, lower alcohols (such as ethanol, isopropanol), hydroalcoholic mixtures, hydrocarbons (such as isobutane, hexane, decene, acetone), halogenated hydrocarbons (such as Freon), linalool, hydrocarbon esters (such as ethyl acetate, dibutyl phthalate), volatile silicon derivatives, especially siloxanes (such as phenyl pentamethyl disiloxane, phenethyl pentamethyl disiloxane, methoxypropyl heptamethyl cyclotetrasiloxane, chloropropyl pentamethyl disiloxane, hydroxypropyl pentamethyl disiloxane, octamethyl cyclotetrasiloxane, decamethyl cyclopentasiloxane), and mixtures thereof. Preferred solvents include water, ethanol, volatile silicon derivatives, and mixtures thereof.

Preferred compositions of the present invention comprise the silicone copolymers, as described *supra*, in combination with silicone conditioning agents as described *supra*.

The unique vehicle systems of the present invention provide superior performance *vis a vis* delivery of the active cosmetic component to the hair or skin. This is especially true in the case of hair care compositions. Lower levels of active components may be used in the hair care compositions of the present invention, than are used in hair care compositions formulated with alternative thickening systems. These deposition benefits are especially noticable in the case of silicone hair conditioning agents. The quantity and quality of silicone deposit from the present unique vehicle systems onto hair results in enhanced hair conditioning.

These active cosmetic care materials are generally present at a level of from 0% to 20%, preferably from 0.1% to 20%, by weight of the cosmetic composition. The 0% level reflects the situation when one of the vehicle component provides the hair care activity to the present compositions. For example, if the vehicle system comprises a water-insoluble quaternary ammonium compound, this material will provide hair conditioning benefits as well. The level of the active cosmetic care material varies depending upon which active material is chosen, the particular cosmetic compositions to be formulated therewith, and the level of benefit desired.

Other optional components that can be added to the cosmetic compositions of the present invention do not provide any direct cosmetic care benefit but instead enhance the composition in some way. Examples of such materials are coloring agents, such as any of the FD&C or D&C dyes; opacifiers, pearlescent aids, such as ethylene glycol distearate or TiO2 coated mica; pH modifiers, such as citric acid, succinic acid, phosphoric acid, sodium hydroxide, and sodium carbonate; perservatives, such as benzyl alcohol, ethyl paraben, propyl paraben, and imidazolidonyl urea; and antioxidants. Such agents generally are used individually at a level of from 0.001% to 10%, preferably from 0.01% to 5%, of the hair care composition.

The vehicle systems and cosmetic compositions of the present invention can be made using conventional formulation and mixing techniques. In one procedure for manufacture, a silicone conditioner, quaternary ammonium surfactant, and at least a portion of the solvent component are premixed prior to the addition of the remaining components. Methods of making various types of cosmetic compositions are described more specifically in the following examples.

The following examples illustrate the present invention.

All parts, percentages, and ratios herein are by weight unless otherwise specified.

Example I

The following is a hair styling rinse composition representative of the present invention.

| Component | Weight % |
|---|---|
| Styling Agent Premix | |
| Silicone Copolymer [1] | 2.00 |
| Phenylpentamethyl disiloxane | 9.00 |
| Xanthan Premix | |
| Xanthan gum | 0.25 |
| DRO $H_2O$ | 25.00 |
| Main Mix | |
| Dihydrogenated tallow-dimethylammonium chloride (DTDMAC) | 0.50 |
| EDTA, disodium salt | 0.10 |
| D.C. 929 [2] | 2.00 |
| Perfume | 0.10 |
| Natrosol Plus CS Grade D-67 [3] | 0.75 |
| Locust bean gum | 0.75 |
| Kathon CG [4] | 0.04 |
| DRO $H_2O$ | q.s. to 100% |

[1] 20/60/20 N,N-dimethylacrylamide/isobutyl methacrylate/PDMS macromer (20,000 MW), polymer molecular weight about 300,000.

[2] Amodimethicone, commercially available from Dow Corning

[3] Hydrophobically modified hydroxethylcellulose having a $C_{16}$ alkyl substitution of from about 0.50% to about 0.95%, by weight, and a hydroxyethyl molar substitution of from about 2.3 to about 3.3, and where the average molecular weight of the hydroxyethyl cellulose prior to substitution is approximately 700,000, available from Aqualon Company.

[4] preservative commercially available from Rohm and Haas

The composition is prepared as follows. The DRO (double reverse osmosis) water is first heated to 88 °C (190 °F). The DTDMAC, EDTA, and D.C. 929 are added to the water and mixed for about 5 minutes. The Natrosol is added to the composition with mixing. The Locust Bean Gum is added to the composition with mixing. The composition is then homogenized with a disperser, for example a Gifford-Wood mill, for about 2 minutes. The batch is then cooled to 66 °C (150 °F). The xanthan gum premix, styling agent premix, perfume and Kathan CG are added to the composition with mixing for about 10 minutes. The batch is cooled to ambient temperature and stored.

Example II

The following is a hair styling rinse composition representative of the present invention.

| Component | Weight % |
|---|---|
| Premix A | |
| Silicone Copolymer [1] | 1.00 |
| DRO $H_2O$ | 10.00 |
| Premix B | |
| Silicone Copolymer [2] | 3.00 |
| DRO $H_2O$ | 15.00 |
| Main Mix | |
| Natrosol Plus CS Grade D-67 [3] | 1.00 |
| Stearamide DEA | 0.50 |
| Ethanol | 10.00 |
| Perfume | 0.20 |
| DRO $H_2O$ | q.s. to 100% |

[1] 40/40/20 quaternized dimethylaminoethyl methacrylate/isobutyl methacrylate/silicone macromer, the macromer having a molecular weight of about 20,000 prepared in a manner similar to Example C-2c of U.S. Patent 4,728,571 polymer molecular weight about 500,000

[2] 40/40/20 acrylic acid/methyl methacrylate/silicone macromer, the macromer having a molecular weight of about 20,000 prepared in a manner similar to Example C-2c of U.S. Patent 4,728,571, 1988, polymer molecular weight about 400,000

[3] hydrophobically-modified hydroxyethyl cellulose, commercially available from Aqualon Co.

The composition is prepared as follows. The DRO water is first heated to 88°C (190°F). The stearamide DEA and Natrosol are added and the composition is mixed for about 5 minutes. The composition is then homogenized with a disperser, for example a Gifford-Wood mill, for about 2 minutes. The composition is cooled to 49°C (120°F) and Premix A, Premix B, ethanol, and perfume are added with mixing for about 10 minutes. The composition is cooled to ambient temperature and stored.

Example III

The following is a hair styling rinse composition representative of the present invention.

| Component | Weight % |
|---|---|
| Styling Agent Premix | |
| Silicone Copolymer [1] | 3.00 |
| Phenylpentamethyl disiloxane | 9.00 |
| Hydroxypropylpentamethyl disiloxane | 6.00 |
| Silicone Gum Premix | |
| Silicone Gum G.E. SE 76 [2] | 0.50 |
| Decamethyl cyclopentasiloxane | 4.00 |
| Main Mix | |
| Natrosol Plus CS Grade D-67 [3] | 0.60 |
| Locust bean gum | 0.50 |
| EDTA, disodium salt | 0.15 |
| DTDMAC | 0.65 |
| Glydant [4] | 0.40 |
| Perfume | 0.20 |
| DRO $H_2O$ | q.s. to 100% |

[1] 10/70/20 acrylic acid/n-butyl methacrylate/silicone macromer, the macromer having a molecular weight of about 20,000, prepared in a manner similar to Example C-2c of U.S. Patent 4,728,571, polymer molecular weight about 300,000

[2] Commercially available from General Electric

[3] hydrophobically-modified hydroxyethyl cellulose commercially available from Aqualon Co.

[4] preservative commercially available from Glyco, Inc.

The composition is prepared as follows. The DRO water is heated to 88 °C (190 °F). The DTDMAC, EDTA, and silicone gum premix are added to the water with mixing for about 5 minutes. The Natrosol is added with mixing. The Locust Bean Gum is added with mixing. The composition is then homogenized with a disperser, for example a Gifford-Wood mill, for about 2 minutes. The batch is cooled to 66 °C (150 °F) and the styling agent premix, the perfume and the Glydant are added with mixing for about 10 minutes. The batch is cooled to ambient temperature and stored.

Example IV

The following is a hair styling rinse composition representative of the present invention.

| Component | Weight % |
|---|---|
| Natrosol Plus CS Grade D-67[1] | 1.20 |
| Xanthan Gum | 0.25 |
| Citric Acid | 0.073 |
| Sodium Citrate | 0.175 |
| Kathon CG | 0.033 |
| DiTallow DiMethyl Ammonium Chloride (DTDMAC) | 0.75 |
| Hydrogenated Tallow Betaine | 0.33 |
| T-Butyl Acrylate/PDMS Copolymer (10,000 MW - 80/20 W/W) | 2.50 |
| Phenethyl Pentamethyl Disiloxane | 1.875 |
| D4 Cyclomethicone | 5.625 |
| Polydimethyl Siloxane Gum/D5 Cyclomethicone Premix (15/85)[2] | 2.333 |
| Perfume | q.s. |
| DRO Water | q.s. to 100% |

[1] Hydrophobically modified hydroxyethyl cellulose available from Aqualon Corp.
[2] G.E. SE-76 gum available from G. E. Silicones

The composition is prepared as follows. The xanthan gum is first slurried in water at 4% xanthan gum, until fully hydrated. In a separate vessel the copolymer is mixed into the phenethyl pentamethyl disiloxane and D4 cyclomethicone.

The remaining water is preheated to about 88°C. The DTDMAC, citric acid, sodium citrate, and hydrogenated tallow betaine are added to the water and mixed until melted. This mixture is then cooled to about 65°C. The Natrosol Plus, silicone gum premix, Kathon and perfume are added and mixed until homogeneous. This mixture is then cooled to about 43°C. The xanthan gum premix and copolymer premix are then added and the mixture is agitated until homogeneous. The resulting composition is cooled to ambient temperature.

Example V

The following is a hand cream composition representative of the present invention.

| Component | Wt.% |
|---|---|
| Natrosol Plus CS Grade D-67 [1] | 1.0 |
| Carboxymethylcellulose | 0.6 |
| Dimethyl Stearamine oxide | 1.0 |
| EDTA, disodium salt | 0.15 |
| Aloe vera | 0.5 |
| Preservative/perfume | 0.3 |
| DRO Water | q.s.to 100% |

[1] Hydrophobically modified hydroxethylcellulose available from Aqualon

All ingredients are combined and mixed at 90°C for about 1/2 hour then cooled to ambient temperature and stored.

Example VI

The following is an antidandruff cream rinse and conditioning composition representative of the present invention.

| Component | Wt. % |
|---|---|
| Natrosol Plus CS Grade D-67 [1] | 1.2 |
| Dimethyl Stearamine oxide | 0.5 |
| Zinc pyrithione | 1.0 |
| Preservative/perfume | 0.3 |
| DRO Water | q.s. to 100% |

[1] Hydrophobically modified hydroxethylcellulose available from Aqualon

The composition is prepared as follows. The DRO water is first heated to 88°C (190°F). The stearamine oxide, and Natrosol are added with mixing. The composition is then homogenized with a disperser, e.g., a Gifford-Wood mill, for several minutes. The batch is cooled to 66°C (150°F). The perfume, preservative, and ZPT are added and the composition is mixed for 10 minutes. The batch is cooled to ambient temperature and stored.

Example VII

The following is a sunscreen composition representative of the present invention.

| Component | Wt. % |
|---|---|
| Natrosol Plus CS Grade D-67 [1] | 1.0 |
| Stearamide DEA | 0.5 |
| PABA | 2.5 |
| Preservative/perfume | 0.3 |
| DRO Water | q.s.to 100% |

[1] Hydrophobically modified hydroxethylcellulose available from Aqualon

The composition is prepared as follows. The DRO water is heated to 88°C (190°F). The Stearamide DEA, and Natrosol are added with mixing. The composition is then homogenized with a disperser, e.g., a Gifford-Wood mill, for several minutes. The batch is cooled to 66°C (150°F). The perfume, preservative, and PABA are added and the composition is mixed for 10 minutes. The batch is cooled to ambient temperatures and stored.

EXAMPLE VIII

The following is a hair tonic composition which is representative of the present invention.

| Component | Wt. % |
|---|---|
| Natrosol Plus CS Grade D-67 [1] | 1.00 |
| Dimethyl Stearamine oxide | 0.20 |
| Mineral Oil | 2.00 |
| Kathon CG | 0.04 |
| Perfume | 0.05 |
| DRO Water | q.s. to 100% |

[1] Hydrophobically modified hydroxethylcellulose available from Aqualon

The composition is prepared as follows. The DRO water is heated to 88°C (190°F). The stearamine oxide is added and mixed for 5 minutes. The mineral oil and Natrosol are added and the composition is homogenized with a disperser, e.g., a T-50 Ultra-turax by Tekmar, for about 2 minutes. The batch is cooled to 66°C (150°F). The perfume and preservative are added and the batch mixed for 10 minutes. The batch

is then cooled to ambient temperature and stored.

Example IX

The following is a hair conditioning rinse composition which is representative of the present invention.

| Component | Wt. % |
|---|---|
| Silicone Gum Premix | |
| Octamethyl Cyclotetrasiloxane | 3.00 |
| G.E. SE 76 [2] | 0.50 |
| Main Mix | |
| Natrosol Plus CS Grade D-67 [1] | 1.25 |
| Dihydrogenated tallow dimethyl ammonium chloride (DTDMAC) | 0.75 |
| Stearamide DEA | 0.10 |
| Kathon CG | 0.04 |
| DRO Water and fragrance | q.s.to 100% |

[1] Hydrophobically modified hydroxyethylcellulose available from Aqualon
[2] Silicone gum available from General Electric

The composition is prepared as follows. The DRO water is first heated to 88°C (190°F). The DTDMAC, stearamide DEA, Natrosol, and the Silicone gum premix are added with mixing. The composition is then homogenized with a disperser, e.g., a Gifford-Wood mill, for about 2 minutes. The composition is cooled to 66°C (150°F) and the Kathon and perfume are added with mixing for about 10 minutes. The batch is cooled to ambient temperature and stored.

Example X

The following is a hair antidandruff tonic composition which is representative of the present invention.

| Component | Wt. % |
|---|---|
| Natrosol Plus CS Grade D-67 [1] | 0.75 |
| Zinc Omadine [2] | 0.05 |
| Ceteth-2 | 0.50 |
| Kathon | 0.04 |
| Perfume | 0.02 |
| DRO Water | q.s.to 100% |

[1] Hydrophobically modified hydroxyethylcellulose available from Aqualon
[2] Antidandruff active available from Olin

The composition is prepared as follows. The DRO water is first heated to 88°C (190°F). The Ceteth-2 and Natrosol are added with mixing for about 5 minutes. The composition is then homogenized with a disperser, e.g., a Gifford-Wood mill, for several minutes. The batch is cooled to 66°C (150°F). The zinc omadine and fragrance are added with mixing for about 10 minutes. The batch is cooled to ambient temperatures and stored.

Example XI

The following is a hair growth tonic composition which is representative of the present invention.

| Component | Wt. % |
|---|---|
| Minoxidil [1] | 1.75 |
| Propylene Glycol | 10.00 |
| Oleyl Alcohol | 1.00 |
| Natrosol Plus CS Grade D-67 [2] | 1.00 |
| Cocamide MEA | 0.30 |
| Kathon | 0.04 |
| Perfume | 0.02 |
| DRO Water | q.s. to 100% |

[1] Hair growth active - available from Upjohn
[2] Hydrophobically modified hydroxyethylcellulose available from Aqualon

The composition is prepared as follows. All ingredients are combined and mixed at 90°C for about 1/2 hour then cooled to ambient temperature and stored.

Example XII

The following is a hair styling conditioner composition which is representative of the present invention.

| Component | Wt. % |
|---|---|
| Disodium EDTA | 0.10 |
| Monosodium Phosphate | 0.08 |
| Disodium Phosphate | 0.02 |
| Tallow Diethanol Amide | 0.60 |
| Natrosol Plus CS Grade D-67 [1] | 1.50 |
| Glydant | 0.37 |
| Perfume | 0.02 |
| DRO Water | q.s.to 100% |
| Styling Polymer Premix | |
| Styling Polymer [2] | 3.00 |
| Phenyl Pentamethyl Disiloxane | 4.95 |
| Octamethyl Cyclotetrasiloxane | 4.05 |
| Silicone Gum Premix | |
| G. E. S E 76 [3] | 0.75 |
| Octamethyl Cyclotetrasiloxane | 4.25 |

[1] Hydrophobically modified hydroxyethylcellulose available from Aqualon
[2] Isobutylmethacrylate/2-ethylhexylmethacrylate/N,N-dimethylacrylamide copolymer 80/5/15
[3] Silicone Gum available from General Electric

The composition is prepared as follows. The DRO water is first heated to 88°C (190°F). The EDTA, tallow diethanolamide, mono- and disodium phosphate are added with mixing for about 5 minutes. The Natrosol is added with mixing. The batch is cooled to 66°C (150°F). The Silicone Gum Premix is added with mixing. The composition is then homogenized using a disperser, e.g., a Gifford-Wood mill, for about 2 minutes. The batch is cooled to 66°C (150°F). The perfume, Styling Polymer Premix and Glydant are

added with mixing for about 10 minutes. The batch is cooled to ambient temperature and stored.

Example XIII

The following is a hair styling conditioner composition which is representative of the present invention.

| Component | Wt. % |
|---|---|
| Disodium EDTA | 0.15 |
| Monosodium Phosphate | 0.04 |
| Disodium Phosphate | 0.12 |
| Dihydrogenated tallow dimethyl ammonium chloride (DTDMAC) | 0.75 |
| Locust Bean Gum | 0.70 |
| Natrosol Plus CS Grade D-67 [1] | 0.70 |
| Glydant | 0.37 |
| Perfume | 0.02 |
| Water | q.s.to 100% |
| Silicone Gum Premix | |
| G. E. S E 76 [2] | 0.50 |
| Octamethyl Cyclotetrasiloxane | 3.00 |
| Xanthan Gum | 0.25 |
| Styling Polymer Premix | |
| Styling Polymer [3] | 3.00 |
| Phenyl Pentamethyl Disiloxane | 9.00 |
| Hydroxypropyl Pentamethyl Disiloxane | 6.00 |

[1] Hydrophobically modified hydroxyethylcellulose available from Aqualon
[2] Silicone Gum available from General Electric
[3] Isobutylmethacrylate/2-ethylhexylmethacrylate/N,N-dimethylacrylamide copolymer 80/5/15

The composition is prepared as follows. The DRO water is heated to 88 °C (190 °F). The DTDMAC, disodium EDTA, monosodium phosphate, and disodium phosphate are added and the composition is mixed for about 5 minutes. The silicone gum premix, locust bean gum, and Natrosol are added with mixing. The composition is then homogenized using a disperser, e.g., a Gifford-Wood Mill, for about 2 minutes. The batch is cooled to 66 °C (150 °F) and the Xanthan Gum premix, styling polymer premix, perfume and Glydant are added and mixed for about 10 minutes. The composition is then cooled to ambient temperature and stored.

Example XIV

The following is a styling rinse composition representative of the present invention.

| Component | Wt. % |
|---|---|
| Styling Agent | |
| Silicone Copolymer[1] | 3.00 |
| Octamethyl cyclotetrasiloxane | 9.00 |
| Premix | |
| Silicone Gum GE SE76[2] | 0.50 |
| Decamethyl cyclopentosiloxane | 4.00 |
| Main Mix | |
| Natrosol Plus CS Grade D-67 [3] | 1.25 |
| Stearamide DEA | 0.40 |
| DTDMAC | 0.50 |
| Kathon CG[4] | 0.03 |
| Imidazole | 0.15 |
| Perfume | 0.10 |
| DRO $H_2O$ | q.s. to 100% |

[1] 80/20 t-butyacrylate/PDMS macromer, the macromer having a molecular weight of about 10,000, prepared in a manner similar to Example C-2b of U.S. Patent 4,728,571.
[2] Commercially available from General Electric
[3] hydrophobically-modified hydroxyethyl cellulose commerically available from Aqualon Co.
[4] preservative commercially available from Rohm & Haas

The composition is prepared as follows. The Styling Agent and Premix are blended separately by conventional means. The Main Mix is prepared by adding all the ingredients and heating to 95 °C for 1/2 hour with agitation. As the batch is cooled to about 60 °C, the Premix and Styling Agent mixes are added to the Main Mix with agitation and the batch is cooled to ambient temperature.

Example XV

The following is a hair styling conditioner composition which is representative of the present invention.

| Ingredient | Wt. % |
|---|---|
| Premix: | |
| G. E. SE 76 Gum [1] | 0.80 |
| Cab-O-Sil HS-5 [2] | 0.20 |
| Decamethylcyclopentasiloxane | 4.50 |
| Natrosol Plus CS Grade D-67 [3] | 1.40 |
| Hydrogenated Tallowamide DEA | 0.58 |
| Adogen 442 - 100P [4] | 0.50 |
| Glydant [5] | 0.37 |
| Disodium EDTA [6] | 0.15 |
| Disodium phosphate | 0.12 |
| Monosodium phosphate | 0.03 |
| P.E.G. 600 | 0.50 |
| Fragrance | 0.02 |
| DRO $H_2O$ | q.s. to 100% |

[1] Polydimethylsiloxane gum offered by General Electric
[2] Fumed silica offered by Cabot Corp.
[3] Hydrophobically modified hydroxyethyl cellulose available from Aqualon
[4] Dihydrogenated tallow dimethyl ammonium chloride offered by Sherex Chemical Co.
[5] Preservative offered by Glyco, Inc.
[6] Ethylene diamine tetraacetic acid

The composition is prepared as follows. The DRO water is heated to 66°C (150°F). The EDTA, PEG, phosphates, DEA, and Adogen are added to the water with mixing for about 10 minutes. The Natrosol is then added with mixing for about 5 minutes. The silicone gum premix is then added with mixing. The composition is then homogenized with a disperser, for example a Gifford-Wood mill, for about 2 minutes. The batch is cooled to 38°C (100°F). The Glydant and fragrance are added with mixing for about 10 minutes. The batch is cooled to ambient temperature and stored.

Example XVI

The following is a hair styling composition which is representative of the present invention.

| Ingredient | Wt. % |
|---|---|
| Premix 1: | |
| G. E. SE 76 Gum [1] | 0.80 |
| Cab-O-Sil HS-5 [2] | 0.20 |
| Decamethylcyclopentasiloxane | 4.50 |
| Premix 2: | |
| G. E. SE 76 Gum | 0.50 |
| Decamethylcyclopentasiloxane | 2.80 |
| Natrosol Plus CS Grade D-67 [3] | 1.39 |
| Hydrogenated tallowamide DEA | 0.56 |
| Adogen 442 - 100P [4] | 0.50 |
| Glydant [5] | 0.37 |
| Disodium phosphate | 0.12 |
| Monosodium phosphate | 0.03 |
| Disodium EDTA [6] | 0.15 |
| Fragrance | 0.02 |
| DRO $H_2O$ | q.s. to 100% |

[1] Polydimethylsiloxane gum offered by General Electric
[2] Fumed silica offered by the Cabot Corp.
[3] Hydrophobically modified hydroxyethyl cellulose available from Aqualon
[4] Dihydrogenated tallow dimethyl ammonium chloride offered by Sherex Chemical Co.
[5] Preservative offered by Glyco, Inc.
[6] Ethylene diamine tetraacetic acid

The composition is prepared as follows. The DRO water is heated to 66°C (150°F). The EDTA, phosphates, DEA, and Adogen are added to the water with mixing for about 10 minutes. The Natrosol is then added with mixing for about 5 minutes. The silicone gum premixes are then added with mixing. The composition is then homogenized with a disperser, for example a Gifford-Wood Mill, for about 2 minutes. The batch is cooled to 38°C (100°F). The Glydant and perfume are added with mixing for about 10 minutes. The batch is cooled to ambient temperature and stored.

Example XVII

The following is a hair conditioner which is representative of the present invention.

| Ingredient | Wt. % |
|---|---|
| Premix: | |
| G. E. SE 76 Gum [1] | 0.10 |
| Decamethylcyclopentasiloxane | 0.60 |
| Natrosol Plus CS Grade D-67 [2] | 1.50 |
| Hydrogenated tallowamide DEA | 0.70 |
| Adogen 442 - 100P [3] | 0.50 |
| Glydant [4] | 0.37 |
| Disodium EDTA [5] | 0.15 |
| Disodium phosphate | 0.12 |
| Monosodium phosphate | 0.03 |
| Fragrance | 0.02 |
| DRO $H_2O$ | q.s. to 100% |

[1] Polydimethylsiloxane gum offered by General Electric
[2] Hydrophobically modified hydroxyethyl cellulose available from Aqualon
[3] Dihydrogenated tallow dimethyl ammonium chloride offered by Sherex Chemical Co.
[4] Preservative offered by Glyco, Inc.
[5] Ethylene diamine tetraacetic acid

The composition is prepared as follows. The DRO water is heated to 66°C (150°F). The EDTA, phosphates, DEA, and Adogen are added to the water with mixing for about 10 minutes. The Natrosol is then added with mixing for about 5 minutes. The silicone gum premix is then added with mixing. The composition is then homogenized with a disperser, for example a Gifford-Wood Mill, for about 2 minutes. The batch is cooled to 38°C (100°F). The Glydant and perfume are added with mixing for about 10 minutes. The batch is cooled to ambient temperature and stored.

Example XVIII

A hair conditioning rinse of the present invention is as follows:

| Component | Weight % |
|---|---|
| Polydimethylsiloxane Gum (G.E. SE-76) | 0.75 |
| Decamethylcyclopentasiloxane | 4.25 |
| Natrosol Plus CS Grade D-67 | 0.95 |
| Quaternium 18 (Adogen 442-100P) | 0.75 |
| Stearamide DEA | 0.44 |
| Stearyl Alcohol | 0.20 |
| Cetyl Alcohol | 0.30 |
| Disodium EDTA | 0.10 |
| Citric Acid Anhydrous | 0.075 |
| Perfume | 0.25 |
| Preservative | 0.033 |
| DRO Water | q.s. to 100% |

The composition is prepared as follows. The Adogen 442 is first combined with a small amount of water and heated to about 77°C with mixing until melted. The premix is then cooled to about 60°C, and the siloxane gum and decamethyl cyclopentasiloxane (which have been premixed) are added. The premix is mixed for an additional 10 minutes.

The remaining water, citric acid, sodium citrate, and EDTA are separately combined and heated with mixing to about 65°C. The cetyl alcohol, stearyl alcohol and stearamide DEA are then added with mixing. The Natrosol Plus is then added with mixing until the composition thickens. The premix is then added with mixing and the composition is cooled to about 50°C. The perfume and Kathon are added and the composition is cooled with milling to ambient temperature.

Example XIX

A hair conditioning rinse of the present invention is as follows:

| Component | Weight % |
|---|---|
| Polydimethylsiloxane Gum | 1.00 |
| Decamethylcyclopentasiloxane | 5.67 |
| Adogen 442-100P (quaternium-18) | 1.00 |
| Natrosol Plus Grade D-67 | 0.80 |
| Stearamide DEA | 0.44 |
| Stearyl Alcohol | 0.30 |
| Cetyl Alcohol | 0.45 |
| Disodium EDTA | 0.10 |
| Sodium Citrate | 0.03 |
| Citric Acid | 0.08 |
| Fragrance | 0.25 |
| Kathon | 0.033 |
| DRO Water | q.s. to 100% |

The composition is prepared as is described in Example XVIII.

Example XX

A hair conditioning rinse of the present invention is as follows:

| Component | Weight % |
|---|---|
| Polydimethylsiloxane Gum | 1.00 |
| Decamethylcyclopentasiloxane | 5.67 |
| Adogen 442-100P (quaternium-18) | 1.00 |
| Natrosol Plus Grade D-67 | 0.80 |
| Stearamide DEA | 0.44 |
| Stearyl Alcohol | 0.40 |
| Cetyl Alcohol | 0.60 |
| Disodium EDTA | 0.10 |
| Sodium Citrate | 0.03 |
| Citric Acid | 0.08 |
| Fragrance | 0.25 |
| Kathon | 0.033 |
| DRO Water | q.s. to 100% |

The composition is prepared as is described in Example XVIII.

Example XXI

The following is a hair styling rinse composition representative of the present invention.

| Component | Weight % |
|---|---|
| Natrosol Plus CS Grade D-67[1] | 1.15 |
| Hydrogenated Tallow Betaine | 0.30 |
| DiTallow DiMethyl Ammonium Chloride (DTDMAC) | 0.75 |
| Citric Acid | 0.07 |
| Sodium Citrate | 0.17 |
| Styling Polymer Premix - | |
| Styling Polymer[2] | 2.5 |
| Phenyl Ethyl Pentamethyl Disiloxane | 1.875 |
| Octamethyl Cyclotetrasiloxane | 5.625 |
| Silicone Gum Premix - | |
| Polydimethyl Siloxane Gum[3] | 0.35 |
| Decamethyl Cyclopentasiloxane | 1.98 |
| Kathon CG | 0.033 |
| Perfume | 0.2 |
| Xanthan Gum[4] | 0.25 |
| DRO Water | q.s. to 100% |

[1] Hydrophobically modified hydroxyethyl cellulose available from Aqualon Corp.
[2] 80.20 t-Butylacrylate/PDMS macromer, the macromer having a molecular weight of about 10,000, prepared in a manner similar to Example C-2b of U.S. Patent 4,728,571, Clemens, issued March 1, 1988
[3] S.E.-76 gum available from General Electric
[4] Readily dispersible xanthan gum

The composition is prepared as follows.

The styling polymer premix is prepared by combining the styling polymer, phenyl ethyl pentamethyl disiloxane, and the octamethyl cyclotetrasiloxane.

The silicone gum premix is prepared by combining, in a separate vessel and mixing the silicone gum and the decamethyl cyclopenta siloxane until homogeneous.

About one-half of the DRO water is first heated to about 66 °C. The hydrogenated tallow betaine, citric acid, and sodium citrate are added and mixed until homogeneous. The Natrosol and xanthan gum are added and mixed until homogeneous. The composition is cooled to about 38 °C. The styling polymer premix, Kathon CG and perfume are added. The composition is mixed and homogenized with a homogenlzer such as a Tekmar homogenizer (preferably in-line).

The remaining DRO water is heated to about 88 °C, the DTDMAC is added and mixed until homogeneous. The mixture is then cooled to about 43 °C. The silicone gum premix is added and the composition homogenized with a homogenizer (in-line preferred).

The two premixes are then combined and mixed until homogeneous to form the styling rinse composition.

Example XXII

The following is a hair styling rinse composition representative of the present invention.

| Component | Weight % |
|---|---|
| Natrosol Plus CS Grade D-67[1] | 1.15 |
| Hydrogenated Tallow Betaine | 0.30 |
| DiTallow DiMethyl Ammonium Chloride (DTDMAC) | 0.75 |
| Stearyl Alcohol | 0.2 |
| Cetyl Alcohol | 0.3 |
| Citric Acid | 0.07 |
| Sodium Citrate | 0.17 |
| Styling Polymer Premix - | |
| Styling Polymer[2] | 2.5 |
| Phenyl Ethyl Pentamethyl Disiloxane | 1.875 |
| Octamethyl Cyclotetrasiloxane | 5.625 |
| Silicone Gum Premix - | |
| Polydimethyl Siloxane Gum[3] | 0.35 |
| Decamethyl Cyclopentasiloxane | 1.98 |
| Kathon CG | 0.033 |
| Perfume | 0.2 |
| Xanthan Gum[4] | 0.25 |
| DRO Water | q.s. to 100% |

[1] Hydrophobically modified hydroxyethyl cellulose available from Aqualon Corp.
[2] 80/20 t-Butylacrylate/PDMS macromer, the macromer having a molecular weight of about 10,000, prepared in a manner similar to Example C-2b of U.S. Patent 4,728,571, Clemens, issued March 1, 1988
[3] S.E.-76 gum available from General Electric
[4] Readily dispersible xanthan gum

The composition is prepared as follows.

The styling polymer premix is prepared by combining the styling polymer, phenyl ethyl pentamethyl disiloxane, and the octamethyl cyclotetrasiloxane.

The silicone gum premix is prepared by combining, in a separate vessel and mixing the silicone gum and the decamethyl cyclopenta siloxane until homogeneous.

About one-half of the DRO water is first heated to about 66°C. The hydrogenated tallow betaine, citric acid, and sodium citrate are added and mixed until homogeneous. The Natrosol and xanthan gum are added and mixed until homogeneous. The composition is cooled to about 38°C. The styling polymer premix, Kathon CG and perfume are added. The composition is mixed and homogenized with a homogenizer such as a Tekmar homogenizer (preferably in-line).

The remaining DRO water is heated to about 88°C, the DTDMAC, stearyl alcohol and cetyl alcohol are added and mixed until homogeneous. The mixture is then cooled to about 43°C. The silicone gum premix is added and the composition homogenized with a homogenizer (in-line preferred).

The two premixes are then combined and mixed until homogeneous to form the styling rinse composition.

Example XXIII

The following is a hair styling rinse composition representative of the present invention.

| Component | Weight % |
|---|---|
| Natrosol Plus CS Grade D-67[1] | 1.15 |
| Hydrogenated Tallow Betaine | 0.30 |
| DiTallow DiMethyl Ammonium Chloride (DTDMAC) | 0.75 |
| Citric Acid | 0.07 |
| Sodium Citrate | 0.17 |
| Styling Polymer Premix - | |
| Styling Polymer[2] | 2.5 |
| Phenyl Ethyl Pentamethyl Disiloxane | 1.875 |
| Octamethyl Cyclotetrasiloxane | 5.625 |
| Silicone Gum/Fluid Premix | |
| Polydimethyl Siloxane Gum[3] | 0.30 |
| 350 centistoke Polydimethyl Siloxane Fluid | 0.20 |
| Kathon CG | 0.033 |
| Perfume | 0.2 |
| Xanthan Gum[4] | 0.25 |
| DRO Water | q.s. to 100% |

[1] Hydrophobically modified hydroxyethyl cellulose available from Aqualon Corp.
[2] 80/20 t-Butylacrylate/PDMS macromer, the macromer having a molecular weight of about 10,000, prepared in a manner similar to Example C-2b of U.S. Patent 4,728,571, Clemens, issued March 1, 1988
[3] S.E.-76 gum available from General Electric
[4] Readily dispersible xanthan gum

The composition is prepared as follows.

The styling polymer premix is prepared by combining the styling polymer, phenyl ethyl pentamethyl disiloxane, and the octamethyl cyclotetrasiloxane.

The silicone gum/fluid premix is prepared by combining in a separate vessel and mixing the silicone gum and silicone fluid until homogeneous.

About one-half of the DRO water is first heated to about 66°C. The hydrogenated tallow betaine, citric acid, and sodium citrate are added and mixed until homogeneous. The Natrosol and xanthan gum are added and mixed until homogeneous. The composition is cooled to about 38°C. The styling polymer premix, Kathon CG and perfume are added. The composition is mixed and homogenized with a homogenizer such as a Tekmar homogenizer (preferably in-line).

The remaining DRO water is heated to about 88°C, the DTDMAC is added and mixed until homogeneous. The mixture is then cooled to about 43°C. The silicone gum/fluid premix is added and the composition homogenized with a homogenizer (in-line preferred).

The two premixes are then combined and mixed until homogeneous to form the styling rinse composition.

Example XXIV

The following is a hair styling rinse composition representative of the present invention.

| Component | Weight % |
|---|---|
| Natrosol Plus - Grade 330 | 2.0 |
| Hydrogenated Tallow Betaine | 0.30 |
| DiTallow DiMethyl Ammonium Chloride (DTDMAC) | 0.75 |
| Citric Acid | 0.07 |
| Sodium Citrate | 0.17 |
| Styling Polymer Premix - | |
| Styling Polymer[2] | 2.5 |
| Phenyl Ethyl Pentamethyl Disiloxane | 1.875 |
| Octamethyl Cyclotetrasiloxane | 5.625 |
| Silicone Gum Premix - | |
| Polydimethyl Siloxane Gum[3] | 0.35 |
| Decamethyl Cyclopentasiloxane | 1.98 |
| Kathon CG | 0.033 |
| Perfume | 0.2 |
| Xanthan Gum[4] | 0.25 |
| DRO Water | q.s. to 100% |

[1] Hydrophobically modified hydroxyethyl cellulose available from Aqualon Corp.
[2] 80/20 t-Butylacrylate/PDMS macromer, the macromer having a molecular weight of about 10,000, prepared in a manner similar to Example C-2b of U.S. Patent 4,728,571, Clemens, issued March 1, 1988
[3] S.E.-76 gum available from General Electric
[4] Readily dispersible xanthan gum

The composition is prepared as follows.

The styling polymer premix is prepared by combining the styling polymer, phenyl ethyl pentamethyl disiloxane, and the octamethyl cyclotetrasiloxane.

The silicone gum premix is prepared by combining, in a separate vessel and mixing the silicone gum and the decamethyl cyclopenta siloxane until homogeneous.

About one-half of the DRO water is first heated to about 66 °C. The hydrogenated tallow betaine, citric acid, and sodium citrate are added and mixed until homogeneous. The Natrosol and xanthan gum are added and mixed until homogeneous. The composition is cooled to about 38 °C. The styling polymer premix, Kathon CG and perfume are added. The composition is mixed and homogenized with a homogenizer such as a Tekmar homogenizer (preferably in-line).

The remaining DRO water is heated to about 88 °C, the DTDMAC is added and mixed until homogeneous. The mixture is then cooled to about 43 °C. The silicone gum premix is added and the composition homogenized with a homogenizer (in-line preferred).

The two premixes are then combined and mixed until homogeneous to form the styling rinse composition.

Example XXV

The following is a hair styling rinse composition representative of the present invention.

| Component | Weight % |
|---|---|
| Natrosol Plus CS Grade D-67[1] | 1.15 |
| Hydrogenated Tallow Betaine | 0.30 |
| DiTallow DiMethyl Ammonium Chloride (DTDMAC) | 0.75 |
| Citric Acid | 0.07 |
| Sodium Citrate | 0.17 |
| Styling Polymer Premix - | |
| Styling Polymer[2] | 2.5 |
| Octamethyl Cyclotetrasiloxane | 5.25 |
| Decamethyl Cyclopentasiloxane | 2.25 |
| Silicone Gum Premix - | |
| Polydimethyl Siloxane Gum[3] | 0.35 |
| Decamethyl Cyclopentasiloxane | 1.98 |
| Kathon CG | 0.033 |
| Perfume | 0.2 |
| Xanthan Gum[4] | 0.25 |
| DRO Water | q.s. to 100% |

[1] Hydrophobically modified hydroxyethyl cellulose available from Aqualon Corp.
[2] 80/20 t-Butylacrylate/PDMS macromer, the macromer having a molecular weight of about 10,000, prepared in a manner similar to Example C-2b of U.S. Patent 4,728,571, Clemens, issued March 1, 1988
[3] S.E.-76 gum available from General Electric
[4] Readily dispersible xanthan gum

The composition is prepared as follows.

The styling polymer premix is prepared by combining the styling polymer, the octamethyl cyclotetrasiloxane, and the decamethyl cyclopentasiloxane.

The silicone gum premix is prepared by combining, in a separate vessel and mixing the silicone gum and the decamethyl cyclopenta siloxane until homogeneous.

About one-half of the DRO water is first heated to about 66 °C. The hydrogenated tallow betaine, citric acid, and sodium citrate are added and mixed until homogeneous. The Natrosol and xanthan gum are added and mixed until homogeneous. The composition is cooled to about 38 °C. The styling polymer premix, Kathon CG and perfume are added. The composition is mixed and homogenized with a homogenizer such as a Tekmar homogenizer (preferably in-line).

The remaining DRO water is heated to about 88 °C, the DTDMAC is added and mixed until homogeneous. The mixture is then cooled to about 43 °C. The silicone gum premix is added and the composition homogenized with a homogenizer (in-line preferred).

The two premixes are then combined and mixed until homogeneous to form the styling rinse composition.

**Claims**

**1.** A cosmetic composition characterized in that it comprises:

(a) from 80% to 100% of a vehicle system which comprises:

(A) from 0.1% to 10.0% by weight of the cosmetic composition of a hydrophobically modified nonionic water-soluble polymer which comprises a water-soluble polymer backbone and hydrophobic groups selected from $C_8$-$C_{22}$ alkyl, aryl alkyl, alkyl aryl groups and mixtures thereof; wherein the ratio of the hydrophilic portion to the hydrophobic portion of the polymer is from 10:1 to 1000:1, which is preferably a nonionic cellulose ether having a sufficient degree of nonionic substitution, selected from methyl, hydroxyethyl, and hydroxypropyl, to cause it to be water-soluble and being further substituted with a long chain alkyl radical having 10 to 24 carbon atoms in an amount between 0.2 weight percent and the amount which renders said cellulose ether less than 1% by weight soluble in water; and

(B) from 0.02% to 10.0% by weight of the cosmetic composition of a water-insoluble surfactant having a molecular weight less than 20,000; and

(C) from 65% to 99% by weight of the cosmetic composition of a compatible solvent; and

(b) from 0% to 20% of an active cosmetic component;

wherein said cosmetic compositions comprise no more than 1.0% of water-soluble surfactants, and most preferably wherein the vehicle system provides a rheology to the cosmetic composition that is characterized by a shear stress of from 0 to 50 pascal over a shear rate range of from 0.04 $sec^{-1}$ to 25 $sec^{-1}$.

**2.** The composition according to Claim 1 wherein the nonionic cellulose ether comprises from 0.2% to 5.0% of the cosmetic composition, and preferably wherein the nonionic cellulose ether comprises the long-chain alkyl radical attached via an ether linkage.

**3.** The composition according to Claim 1 or 2 wherein the nonionic cellulose ether is selected from a water-soluble hydroxypropyl cellulose substituted with a long-chain alkyl radical having 10 to 24 carbon atoms in an amount between 0.2 weight percent and the amount which renders the hydroxypropyl cellulose less than 1% by weight soluble in water; and a water-soluble hydroxyethyl cellulose, which preferably has a molecular weight of from 50,000 to 700,000, which is substituted with a long-chain alkyl radical having 10 to 24 carbon atoms in an amount between 0.2 weight percent and the amount which renders the hydroxyethyl cellulose less than 1% by weight soluble in water.

**4.** The composition according to any of Claims 1-3 wherein the water-soluble hydroxyethyl cellulose is substituted with a long chain alkyl radical having 16 carbon atoms in an amount between 0.40% to 0.95%, by weight; the hydroxyethyl molar substitution is from 2.3 to 3.7; and the average molecular weight of the unsubstituted cellulose is from 300,000 to 700,000.

**5.** The composition according to any of Claims 1-4 wherein the water-insoluble surfactant is at a level of from 0.05% to 3.0% and is selected from stearamide DEA, cocamide MEA, dimethyl stearamine oxide, glyceryl monooleate, sucrose stearate, PEG-2 stearamine, Ceteth-2, glycerol stearate citrate, dihydrogenated tallow dimethyl ammonium chloride, Poloxamer 181, hydrogenated tallow dimethyl betaine, hydrogenated tallow amide DEA, and mixtures thereof.

**6.** The composition of Claim 3 which additionally comprises from 0.3% to 5.0% of a water-soluble polymeric material having a molecular weight greater than 20,000, which preferably is selected from hydroxyethyl cellulose, hydroxypropyl cellulose, hydroxypropyl methyl cellulose, polyethylene glycol, polyacrylamide, polyacrylic acid, polyvinyl alcohol, polyvinyl pyrrolidone, dextran, carboxymethylcellulose, acacia plant exudate, ghatti plant exudate, tragacanth plant exudate, sodium alginate, propylene glycol alginate, sodium carrageenan, natural polysaccharides, and mixtures thereof, and which is most preferably a natural polysaccharide, selected from guar gum, locust bean gum, xanthan gum, and mixtures thereof.

**7.** The composition according to any of Claims 1-6 which additionally comprises from 0.05% to 1.0%, by weight of the composition of a chelating agent, which is preferably selected from ethylene diamine tetracetic acid and salts thereof, nitrilo acetic acid and salts thereof, hydroxyethylene diamine triacetic acid and salts thereof, diethylene triamine penta-acetic acid and salts thereof, diethanol glycine and salts thereof, ethanol diglycine and salts thereof, citric acid and salts thereof, phosphoric acid and salts thereof.

**8.** The composition according to any of Claims 1-7 wherein from 0.02% to 2.5% of the water-soluble polymer is selected from water-soluble polymeric materials having a molecular weight greater than 1,000,000, and water-soluble polymeric materials having strong ionic character.

**9.** The cosmetic composition according to any of Claims 1-8 which is a hair care composition which preferably comprises no more than 1% of fatty alcohol materials, wherein said active cosmetic component comprises an active hair care component, which is preferably selected from conditioning

agents, antidandruff aids, hair growth promoters, perfumes, dyes, pigments, hair holding polymers, and mixtures thereof, and most preferably is selected from a volatile silicone fluid having a viscosity of less than 10 centipoise; a non-volatile silicone fluid having a viscosity of less than 100,000 CP; a silicone gum having a viscosity greater than 1,000,000 CP, which is preferably selected from polydimethylsiloxane gums and polyphenylmethylsiloxane gums; and mixtures thereof.

**10.** The composition according to Claim 9 wherein the active hair care component comprises from 0.01% to 10% of a rigid silicone polymer having a complex viscosity of at least 2 x $10^5$ poise, which is preferably selected from organic substituted siloxane gums, silicone elastomers, filler reinforced polydimethyl siloxane gums, resin reinforced siloxanes and crosslinked siloxane polymers; and a volatile carrier for the rigid silicone polymer, which is preferably a cyclic silicone containing from 3 to 7 silicon atoms.

**11.** The composition according to Claim 9 wherein the active hair care component comprises from 0.1% to 10.0% of a copolymer which has a vinyl polymeric backbone having grafted to it monovalent siloxane polymeric moieties, said copolymer comprising C monomers and components selected from A monomers, B monomers, and mixtures thereof, wherein:
A is at least one free radically polymerizable vinyl monomer, the amount by weight of A monomer, when used, being up to 98%, preferably from 5% to 98%, by weight of the total weight of all monomers in said copolymer;
B is at least one reinforcing monomer copolymerizable with A, the amount by weight of B monomer, when used, being up to 98% of the total weight of all monomers in said copolymer, said B monomer being selected from polar monomers and macromers; and
C is a polymeric monomer having a molecular weight of from 1,000 to 50,000 and the general formula

$X(Y)_nSi(R)_{3-m}(Z)_m$

wherein
X is a vinyl group copolymerizable with the A and B monomers
Y is a divalent linking group
R is a hydrogen, lower alkyl, aryl or alkoxy
Z is a monovalent siloxane polymeric moiety having a number average molecular weight of at least 500, is essentially unreactive under copolymerization conditions, and is pendant from said vinyl polymeric backbone after polymerization
n is 0 or 1
m is an integer from 1 to 3
wherein C comprises from 0.01% to 50%, preferably from 0.1% to 50%, of the copolymer.

**12.** The composition according to Claim 11 wherein the active hair treatment component comprises a lipophilic low polarity free radically polymerizable vinyl monomer (A), a hydrophilic polar monomer which is copolymerizable with A (B), and a silicone-containing macromer having a weight average molecular weight of from 1,000 to 50,000 based on polydimethylsiloxane selected from

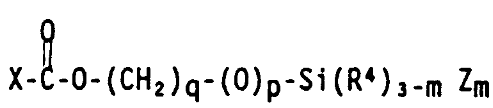

$X\text{-}Si(R^4)_{3-m}\ Z_m$

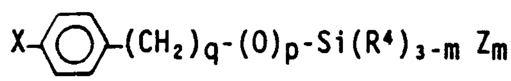

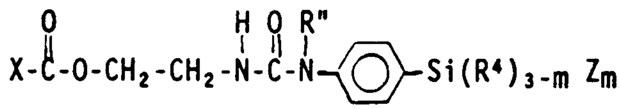

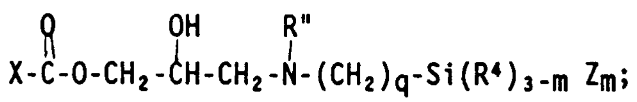 and

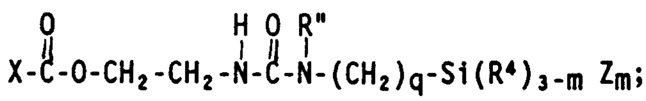

wherein m is 1, 2 or 3; p is 0 or 1; R″ is alkyl or hydrogen; q is an integer from 2 to 6; s is an integer from 0 to 2; X is

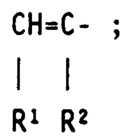

$R^1$ is hydrogen or -COOH; $R^2$ is hydrogen, methyl or $-CH_2COOH$; Z is

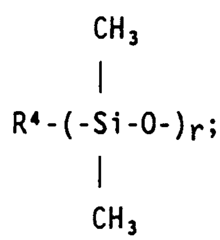

$R^4$ is alkyl, alkoxy, alkylamino, aryl, or hydroxyl; and r is an integer from 5 to 700.

**13.** A hair care composition according to Claim 11 or 12 wherein monomer A is selected from acrylic acid esters of $C_1$-$C_{18}$ alcohols, methacrylic acid esters of $C_1$-$C_{18}$ alcohols, styrene, vinyl acetate, vinyl chloride, vinylidene chloride, acrylonitrile, alpha-methylstyrene, t-butylstyrene, butadiene, cyclohexadiene, ethylene, propylene, vinyl toluene, polystyrene macromer, and mixtures thereof; and is preferably selected from n-butylmethacrylate, isobutylmethacrylate, 2-ethylhexyl methacrylate, t-butylacrylate, t-butylmethacrylate, methylmethacrylate, and mixtures thereof, and wherein monomer B is selected from acrylic acid, methacrylic acid, N,N-dimethylacrylamide, dimethylaminoethyl methacrylate, quaternized dimethylaminoethyl methacrylate, methacrylonitrile, methacryloamide, maleic anhydride, half esters of maleic anhydride, itaconic acid, acrylamide, acrylate alcohols, hydroxyethyl methacrylate, diallyldimethyl ammonium chloride, vinyl pyrrolidone, vinyl ethers, maleimides, vinyl pyridine, vinyl imidazole, styrene sulfonate, and mixtures thereof, and is preferably selected from acrylic acid, N,N-dimethylacrylamide, dlmethylaminoethyl methacrylate, quaternized dimethylaminoethyl methacrylate, vinyl pyrrolidone, and mixtures thereof.

**14.** A hair care composition according to any of Claims 11-13 wherein monomer C has the formula

$$X - \overset{\overset{\displaystyle O}{\|}}{C} - O - (CH_2)_q - (O)_p - Si(R_4)_{3-m}Z_m,$$

preferably wherein p = 0 and q = 3, and most preferably wherein m is 1, r is 250, $R^4$ is alkyl, $R^1$ is hydrogen, and $R^2$ is methyl.

**15.** A hair care composition according to any of Claims 11-14 wherein the silicone-containing copolymer is selected from
acrylic acid/n-butylmethacrylate/polydimethylsiloxane macromer - 20,000 mw (10/70/20);
N,N-dimethylacrylamide/isobutyl methacrylate/PDMS macromer - 20,000 mw (20/60/20);
dimethylaminoethyl methacrylate/isobutyl methacrylate/2-ethylhexyl methacrylate/PDMS - 20,000 mw (25/40/15/20);
dimethylaminoethyl methacrylate/isobutyl methacrylate/PDMS - 20,000 mw (10/70/20);
quaternized dimethylaminoethyl methacrylate/isobutyl methacrylate/PDMS - 20,000 mw (40/40/20);
acrylic acid/methyl methacrylate/PDMS - 20,000 mw (40/40/20);
acrylic acid/isopropyl methacrylate/PDMS - 20,000 mw (25/65/10);
N,N-dimethylacrylamide/methoxyethyl methacrylate/PDMS - 20,000 mw (60/25/15);
dimethylacrylamide/PDMS macromer - 20,000 mw (80/20);
t-butylacrylate/t-butylmethacrylate/PDMS macromer - 10,000 mw (56/24/20);
t-butylacrylate/PDMS macromer - 10,000 mw (80/20);
t-butylacrylate/N,N-dimethylacrylamide/PDMS macromer - 10,000 mw (70/10/20);
t-butylacrylate/acrylic acid/PDMS macromer-10,000 mw (75/5/20); and mixtures thereof.

**16.** A cosmetic composition according to any of Claims 1 to 5 which is a hair care composition wherein the composition comprises:
(a) from 80% to 99.9% of the vehicle system which comprises:
(A) from 0.2% to 5.0%, by weight of the hair care composition, of a nonionic cellulose ether having a hydroxyethyl molar substitution of from 2.3% to 3.7%, and being further substituted with a $C_{16}$ alkyl group at from 0.40% to 0.95%, by weight, wherein the unsubstituted hydroxyethyl cellulose has an average molecular weight of from 300,000 to 700,000;
(B) from 0.05% to 3.0%, by weight of the hair care composition, of the water-insoluble surfactant, wherein the surfactant is selected from stearamide DEA, cocamide MEA, dimethyl stearamine oxide, glyceryl monooleate, sucrose stearate, PEG-2 stearamine, Ceteth-2, glycerol stearate citrate, dihydrogenated tallow dimethyl ammonium chloride, Poloxamer 181, hydrogenated tallow dimethyl betaine, hydrogenated tallow amide DEA, and mixtures thereof;
(C) from 0.05% to 0.3% of a chelating agent which is selected from ethylene diamine tetra acetic acid and salts thereof, citric acid and salts thereof, and phosphoric acid and salts thereof;
(D) from 0.05% to 1.0% of a distributing aid which is selected from xanthan gum and dextran having a molecular weight greater than 1,000,000; and
(b) from 0.1% to 20% of the active cosmetic component which is a hair care component selected from
(A) a silicone-containing copolymer selected from
acrylic acid/n-butylmethacrylate/polydimethylsiloxane macromer - 20,000 mw (10/70/20);
N,N-dimethylacrylamide/isobutyl methacrylate/PDMS macromer - 20,000 mw (20/60/20);
dimethylaminoethyl methacrylate/isobutyl methacrylate/2-ethylhexyl methacrylate/PDMS - 20,000 mw (25/40/15/20);
dimethylaminoethyl methacrylate/isobutyl methacrylate/PDMS - 20,000 mw (10/70/20);
quaternized dimethylaminoethyl methacrylate/isobutyl methacrylate/PDMS - 20,000 mw (40/40/20);
acrylic acid/methyl methacrylate/PDMS - 20,000 mw (40/40/20);
acrylic acid/isopropyl methacrylate/PDMS - 20,000 mw (25/65/10);
N,N-dimethylacrylamide/methoxyethyl methacrylate/PDMS - 20,000 mw (60/25/15);
dimethylacrylamide/PDMS macromer - 20,000 mw (80/20);
t-butylacrylate/t-butylmethacrylate/PPMS macromer - 10,000 mw (56/24/20);

t-butylacrylate/PDMS macromer - 10,000 mw (80/20);
t-butylacrylate/N,N-dimethylacrylamide/PDMS macromer - 10,000 mw (70/10/20);
t-butylacrylate/acrylic acid/PDMS macromer - 10,000 mw (75/5/20); and mixtures thereof, and
(B) a silicone conditioning agent which is selected from
a conditioning agent comprising:
(a) from 0.1% to 2.5% of a polydimethyl siloxane gum;
(b) from 0.02% to 0.7% of fumed silica; and
(c) from 0.4% to 18% of a volatile silicone carrier;
a conditioning agent comprising:
(a) a volatile silicone fluid having a viscosity of less than 10 centipoise;
(b) from 0.5% to 2.0% of a silicone gum having a viscosity of greater than 1,000,000 centipoise;
at ratios of volatile fluid to gum of from 85:15 to 50:50; and
a conditioning agent comprising:
(a) a non-volatile silicone fluid having a viscosity of less than 100,000 centipoise;
(b) from 0.5% to 2.0% of a silicone gum having a viscosity of greater than 1,000,000 centipoise;
at ratios of non-volatile fluid to gum of from 60:40 to 40:60,

wherein said hair care composition comprises no more than 0.5% of water-soluble surfactants; no more than 1% of fatty alcohol materials; and wherein said hair care composition has a rheology that is characterized by a shear stress of from 0 to 50 pascal over a shear rate range of from 0.04 $sec^{-1}$ to 25 $sec^{-1}$.

**17.** A cosmetic composition according to Claim 1 which is a hair conditioning composition wherein the composition comprises:

(a) from 80% to 99.9% of the vehicle system which comprises:

(A) from 0.1% to 10.0%, by weight of the hair conditioning composition, of a nonionic cellulose ether having a sufficient degree of nonionic substitution, selected from methyl, hydroxyethyl, and hydroxypropyl to cause it to be water-soluble and being further substituted with a long chain alkyl radical having 10 to 24 carbon atoms in an amount between 0.2 weight percent and the amount which renders said cellulose ether less than 1% by weight soluble in water, and which is preferably from 0.2% to 5.0% of a hydroxyethyl cellulose substituted with a long chain alkyl radical having 16 carbon atoms in an amount between 0.50% to 0.95%, by weight; the hydroxyethyl molar substitution is from 2.3 to 3.7; and the average molecular weight of the unsubstituted cellulose is from 300,000 to 700,000;

(B) from 0.02% to 10.0%, preferably from 0.05% to 3.0%, by weight of the hair conditioning composition, of a water-insoluble surfactant, having a molecular weight less than 20,000 which is preferably selected from stearamide DEA, cocamide MEA, dimethyl stearamine oxide, glyceryl monooleate, sucrose stearate, PEG-2 stearamine, Ceteth-2, glycerol stearate citrate, Poloxamer 181, hydrogenated tallow dimethyl betaine, hydrogenated tallow amide DEA, and mixtures thereof, and which most preferably comprises hydrogenated tallowamide DEA; and

(b) from 0.1% to 20% of the active cosmetic component which is a hair care component comprising;

(A) from 0.1% to 18%, by weight of the hair conditioning composition, of a silicone conditioning agent which is preferably selected from a volatile silicone fluid having a viscosity of less than 10 centipoise; a non-volatile silicone fluid having a viscosity of less than 100,000 centipoise; a silicone gum having a viscosity greater than 1,000,000 centipoise, which is preferably selected from polydimethylsiloxane gums and polyphenylmethylsiloxane gums; and

(B) up to 1%, by weight of the hair conditioning composition, of a fatty alcohol which is preferably selected from stearyl alcohol, cetyl alcohol, myristyl alcohol, behenyl alcohol, lauryl alcohol, oleyl alcohol, and mixtures thereof, and which is most preferably selected from cetyl alcohol, stearyl alcohol, and mixtures thereof;

wherein a quaternary ammonium compound comprises at least a portion of the water-insoluble surfactant at a level up to 2.5% of the conditioning composition.

**18.** The composition according to Claim 17 wherein the quaternary ammonium compound hair conditioning agent comprises from 0.5% to 2% of di(hydrogenated) tallow dimethyl ammonium chloride.

**19.** The composition according to Claim 17 or 18 additionally comprising from 0.05% to 1.0% of a chelating agent which is selected from ethylene diamine tetracetic acid and salts thereof, nitrilo triacetic acid and salts thereof, hydroxyethylene diamine triacetic acid and salts thereof, diethylene triamine penta-acetic acid and salts thereof, diethanol glycine and salts thereof, ethanol diglycine and salts thereof, citric acid and salts thereof, phosphoric acid and salts thereof.

**20.** The composition according to any of Claims 17-19 wherein the silicone conditioning agent is selected from a combination of a non-volatile silicone fluid having a viscosity of less than 100,000 CP, and from 0.015% to 9.0% of a silicone gum having a viscosity greater than 1,000,000 CP, at a ratio of non-volatile fluid to gum of from 70:30 to 30:70; and a combination of a volatile silicone fluid having a viscosity of less than 10 CP, and from 0.015% to 9.0% of a silicone gum having a viscosity greater than 1,000,000 CP, at a ratio of volatile fluid to gum of from 90:10 to 10:90.

**21.** The composition according to any of Claims 17-20 which comprises up to 1.0% of a trimethylsilylamodimethicone as at least a portion of the silicone conditioning agent.

**22.** The composition according to any of Claims 17-21 wherein a stearamidopropyldimethyl amine comprises at least a portion of the water-insoluble surfactant component at a level up to 1% of the conditioning composition.

**23.** The composition according to any of Claims 17-22 which additionally comprises from 0.1% to 1.5% of a hydrolyzed animal protein.

**24.** A cosmetic composition according to any of Claims 1 to 5 which is a hair conditioning composition wherein the composition comprises:
(a) from 80% to 99.9% of the vehicle system which comprises:
(A) from 0.2% to 5.0%, by weight of the hair conditioning composition, of a nonionic hydroxy ethyl cellulose ether substituted with a long chain alkyl radical having 16 carbon atoms in an amount between 0.50% to 0.95%, by weight; a hydroxyethyl molar substitution of from 2.3 to 3.7; and an average molecular weight of unsubstituted cellulose of from 300,000 to 700,000;
(B) from 0.05% to 3.0%, by weight of the hair conditioning composition, of hydrogenated tallow amide DEA;
(C) from 0.05% to 0.3%, by weight of the hair conditioning composition of a chelating agent selected from ethylene diamine tetracetic acid, and salts thereof; citric acid, and salts thereof; and mixtures thereof; wherein the compatible solvent is water; and
(b) from 0.1% to 20% of an active hair care component comprising;
(A) from 0.5% to 15%, by weight of the hair conditioning composition, of a silicone conditioning agent which is selected from a combination of a volatile silicone fluid having a viscosity of less than 10 centipoise, and from 0.5% to 2.0% of a silicone gum having a viscosity of greater than 1,000,000 centipoise, at ratios of volatile fluid to gum of from 85:15 to 50:50; and a combination of a non-volatile silicone fluid having a viscosity of less than 100,000 centipoise, and from 0.5% to 2.0% of a silicone gum having a viscosity of greater than 1,000,000 centipoise, at ratios of non-volatile fluid to gum of from 60:40 to 40:60; and
(B) from 0.5% to 2.0%, by weight of the hair conditioning composition, of dihydrogenated tallow dimethyl ammonium chloride; and
(C) up to 1%, by weight of the hair conditioning composition, of a fatty alcohol selected from cetyl alcohol, stearyl alcohol, and mixtures thereof;
wherein said hair conditioning composition comprises no more than 0.5% of water-soluble surfactants.

**25.** A method for manufacturing a hair conditioning composition according to any of Claims 17-24 comprising the step of premixing the silicone conditioning agent, the quaternary ammonium compound, and at least a portion of the solvent prior to mixing with the remaining components.

**Patentansprüche**

**1.** Kosmetische Zusammensetzung, dadurch gekennzeichnet, daß sie:
(a) 80% bis 100% eines Trägersystems, enthaltend:

(A) 0,1% bis 10,0%, bezogen auf das Gewicht der kosmetischen Zusammensetzung, von einem hydrohob modifizierten nichtionischen wasserlöslichen Polymer, welches ein wasserlösliches Polymerrückgrat und unter $C_8$-$C_{22}$-Alkyl-, Arylalkyl-, Alkylarylgruppen und Gemischen hievon ausgewählte hydrophobe Gruppen aufweist; worin das Verhältnis vom hydrophilen Anteil zum hydrophoben Anteil des Polymers von 10:1 bis 1000:1 beträgt, welches vorzugsweise ein nichtionischer Celluloseether ist, der einen Grad an unter Methyl, Hydroxyethyl und Hydroxypropyl ausgewählter nichtionischer Substitution aufweist, welcher ausreichend ist, um die Wasserlöslichkeit hervorzurufen, und welcher ferner mit einem langkettigen Alkylrest mit 10 bis 24 Kohlenstoffatomen in einer Menge substituiert ist, die von 0,2 Gew.-% bis zu der Menge reicht, welche die Löslichkeit des genannten Celluloseethers in Wasser auf weniger als 1 Gew.-% verringert; und

(B) 0,02% bis 10,0%, bezogen auf das Gewicht der kosmetischen Zusammensetzung, von einem wasserunlöslichen grenzflächenaktiven Mittel mit einem Molekulargewicht von weniger als 20.000; und

(C) 65% bis 99%, bezogen auf das Gewicht der kosmetischen Zusammensetzung, von einem verträglichen Lösungsmittel;

und

(b) 0% bis 20% einer wirksamen kosmetischen Komponente umfaßt;

worin die genannten kosmetischen Zusammensetzungen nicht mehr als 1,0% an wasserlöslichen grenzflächenaktiven Mitteln enthalten und worin das Trägersystem in der kosmetischen Zusammensetzung im höchsten Maße bevorzugt ein Fließverhalten hervorruft, welches durch eine Scherspannung von 0 bis 50 Pa über einen Schergeschwindigkeitsbereich von 0,04 $s^{-1}$ bis 25 $s^{-1}$ gekennzeichnet ist.

**2.** Zusammensetzung nach Anspruch 1, worin der nichtionische Celluloseether 0,2% bis 5,0% der kosmetischen Zusammensetzung darstellt und worin der nichtionische Celluloseether den langkettigen Alkylrest vorzugsweise über eine Etherbindung gebunden umfaßt.

**3.** Zusammensetzung nach Anspruch 1 oder 2, worin der nichtionische Celluloseether unter einer wasserlöslichen Hydroxypropylcellulose, welche mit einem langkettigen Alkylrest mit 10 bis 24 Kohlenstoffatomen in einer Menge substituiert ist, welche von 0,2 Gew.-% bis zu der Menge reicht, welche die Löslichkeit der Hydroxypropylcellulose in Wasser auf weniger als 1 Gew.-% verringert; und einer wasserlöslichen Hydroxyethylcellulose ausgewählt ist, welche mit einem langkettigen Alkylrest mit 10 bis 24 Kohlenstoffatomen in einer Menge substituiert ist, welche von 0,2 Gew.-% bis zu der Menge reicht, welche die Löslichkeit der Hydroxyethylcellulose in Wasser auf weniger als 1 Gew.-% verringert.

**4.** Zusammensetzung nach einem der Ansprüche 1 bis 3, worin die wasserlösliche Hydroxyethylcellulose mit einem langkettigen Alkylrest mit 16 Kohlenstoffatomen in einer Menge von 0,40 Gew.-% bis 0,95 Gew.-% substituiert ist; die molare Hydroxyethylsubstitution von 2,3 bis 3,7 beträgt; und das mittlere Molekulargewicht der unsubstituierten Cellulose von 300.000 bis 700.000 ist.

**5.** Zusammensetzung nach einem der Ansprüche 1 bis 4, worin das wasserunlösliche grenzflächenaktive Mittel in einer Menge von 0,05% bis 3,0% vorliegt und unter Stearamid-DEA, Kokosamid-MEA, Dimethylstearaminoxid, Glycerylmonooleat, Saccharosestearat, PEG-2-Stearamin, Ceteth-2, Glycerinstearatcitrat, hydriertem Ditalgdimethylammoniumchlorid, Poloxamer 181, hydriertem Talgdimethylbetain, hydriertem Talgamid-DEA und Gemischen hievon ausgewählt ist.

**6.** Zusammensetzung nach Anspruch 3, welche zusätzlich 0,3% bis 5,0% eines wasserlöslichen Polymermaterials mit einem Molekulargewicht von mehr als 20.000 umfaßt, welches vorzugsweise unter Hydroxyethylcellulose, Hydroxypropylcellulose, Hydroxypropylmethylcellulose, Polyethylenglykol, Polyacrylamid, Polyacrylsäure, Polyvinylalkohol, Polyvinylpyrrolidon, Dextran, Carboxymethylcellulose, Akazienpflanzenexsudat, Ghattipflanzenexsudat, Tragantpflanzenexsudat, Natriumalginat, Propylenglykolalginat, Natriumcarageenan, natürlichen Polysacchariden und Gemischen hievon ausgewählt ist, und welches am stärksten bevorzugt ein unter Guargummi, Johannisbrotkernmehl, Xanthangummi und Gemischen hievon ausgewähltes natürliches Polysaccharid ist.

**7.** Zusammensetzung nach einem der Ansprüche 1 bis 6, welche zusätzlich 0,05% bis 1,0%, bezogen auf das Gewicht der Zusammensetzung, von einem Chelatbildner umfaßt, welcher vorzugsweise unter Ethylendiamintetraessigsäure und den Salzen hievon, Nitriloessigsäure und den Salzen hievon, Hydroxyethylendiamintriessigsäure und den Salzen hievon, Diethylentriaminpentaessigsäure und den Salzen hievon, Diethanolglycin und Salzen hievon, Ethanoldiglycin und den Salzen hievon, Zitronensäure und Salzen hievon, Phosphorsäure und den Salzen hievon ausgewählt ist.

**8.** Zusammensetzung nach einem der Ansprüche 1 bis 7, worin 0,02% bis 2,5% des wasserlöslichen Polymers unter wasserlöslichen Polymermaterialien mit einem Molekulargewicht von mehr als 1,000.000 und wasserlöslichen Polymermaterialien mit einem starken ionischen Charakter ausgewählt sind.

**9.** Kosmetische Zusammensetzung nach einem der Ansprüche 1 bis 8, welche eine Haarpflegezusammensetzung ist, die vorzugsweise nicht mehr als 1% Fettalkoholmaterialien enthält, worin die genannte wirksame kosmetische Komponente eine wirksame Haarpflegekomponente umfaßt, welche vorzugsweise unter Konditionierungsmitteln, Antischuppenmitteln, Mitteln zur Förderung des Haarwachstums, Parfums, Farbstoffen, Pigmenten, haarfestigenden Polymeren und Gemischen hievon ausgewählt ist, und am stärksten bevorzugt unter einem flüchtigen Silikonöl mit einer Viskosität von weniger als 10 cP; einem nicht-flüchtigen Silikonöl mit einer Viskosität von weniger als 100.000 cP; einem Silikonkautschuk mit einer Viskosität von mehr als 1,000.000 cP, welcher vorzugsweise unter Polydimethylsiloxankautschuken und Polyphenylmethylsiloxankautschuken ausgewählt ist; und Gemischen hievon ausgewählt ist.

**10.** Zusammensetzung nach Anspruch 9, worin die wirksame Haarpflegekomponente 0,01% bis 10% eines rigiden Silikonpolymers mit einer komplexen Viskosität von mindestens $2x10^5$ Poise, welches vorzugsweise unter organisch substituierten Siloxankautschuken, Silikonelastomeren, mit Füllstoffen verstärkten Polydimethylsiloxankautschuken, mit Harzen verstärkten Siloxanen und vernetzten Siloxanpolymeren ausgewählt ist; und einen flüchtigen Träger für das rigide Silikonpolymer umfaßt, welcher vorzugsweise ein cyclisches Silikon mit 3 bis 7 Siliciumatomen ist.

**11.** Zusammensetzung nach Anspruch 9, worin die wirksame Haarpflegekomponente 0,1% bis 10,0% eines Copolymers enthält, welches ein polymeres Vinylrückgrat aufweist, das darauf gepfropfte einwertige polymere Siloxanreste besitzt, welches Copolymer C-Monomere und unter A-Monomeren, B-Monomeren und Gemischen hievon ausgewählte Komponenten umfaßt, worin:

A mindestens ein freiradikalisch polymerisierbares Vinylmonomer ist, wobei die auf das Gewicht bezogene Menge an A-Monomer, wenn dieses verwendet wird, bis zu 98 Gew.-%, vorzugsweise 5 Gew.-% bis 98 Gew.-%, bezogen auf das Gesamtgewicht aller Monomeren im genannten Copolymer, darstellt;

B mindestens ein mit A copolymerisierbares verstärkendes Monomer ist, wobei die auf das Gewicht bezogene Menge an B-Monomer, wenn dieses verwendet wird, bis zu 98%, bezogen auf das Gesamtgewicht aller Monomeren im genannten Copolymer beträgt, welches B-Monomer unter polaren Monomeren und Makromeren ausgewählt ist; und

C ein polymeres Monomer mit einem Molekulargewicht von 1.000 bis 50.000 und der allgemeinen Formel

$$X(Y)_nSi(R)_{3-m}(Z)_m$$

ist, worin X eine mit den A- und B-Monomeren copolymerisierbare Vinylgruppe darstellt, Y eine zweiwertige verbindende Gruppe bedeutet, R für Wasserstoff, Niederalkyl, Aryl oder Alkoxy steht, Z ein einwertiger polymerer Siloxanrest mit einem Zahlenmittel-Molekulargewicht von mindestens 500 ist, welcher unter Copolymerisationsbedingungen im wesentlichen unreaktiv ist und nach der Polymerisation seitenständig zum genannten polymeren Vinylrückgrat ist, n 0 oder 1 beträgt, m eine ganze Zahl von 1 bis 3 ist,

worin C 0,01% bis 50%, vorzugsweise 0,1% bis 50% des Copolymers darstellt.

**12.** Zusammensetzung nach Anspruch 1, worin die wirksame Haarbehandlungskomponente ein lipophiles freiradikalisch polymerisierbares Vinylmonomer (A) mit einer geringen Polarität, ein hydrophiles polares Monomer, welches mit A copolymerisierbar ist, (B), und ein Silikon enthaltendes Makromer mit einem Gewichtsmittel-Molekulargewicht von 1.000 bis 50.000 umfaßt, das Makromer auf einem Polydimethylsiloxan basiert, welches unter

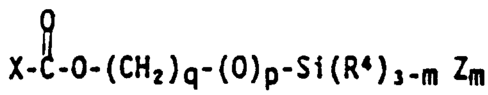

$X\text{-}Si(R^4)_{3-m}\ Z_m$

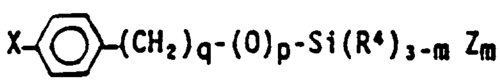

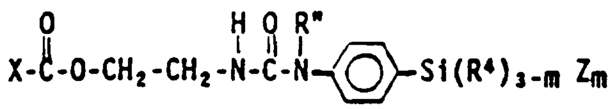

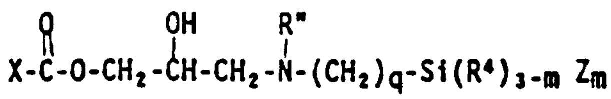

und

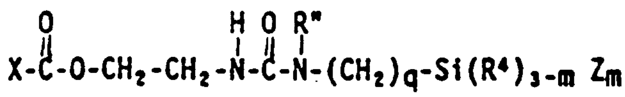

ausgewählt ist, worin m 1, 2 oder 3 beträgt; p 0 oder 1 ist; R'' Alkyl oder Wasserstoff bedeutet; q eine ganze Zahl von 2 bis 6 darstellt; s für eine ganze Zahl von 0 bis 2 steht; X

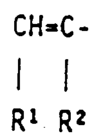

bedeutet, $R^1$ Wasserstoff oder -COOH darstellt; $R^2$ Wasserstoff, Methyl oder $-CH_2COOH$ ist; Z für

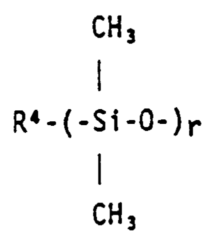

steht; $R^4$ Alkyl, Alkoxy, Alkylamino, Aryl oder Hydroxyl bedeutet; und r eine ganze Zahl von 5 bis 700 ist.

**13.** Haarpflegezusammensetzung nach Anspruch 11 oder 12, worin das Monomer A unter Acrylsäureestern von $C_1$-$C_{18}$-Alkoholen, Methacrylsäureestern von $C_1$-$C_{18}$-Alkoholen, Styrol, Vinylacetat, Vinylchlorid, Vinylidenchlorid, Acrylnitril, alpha-Methylstyrol, tert.Butylstyrol, Butadien, Cyclohexadien, Ethylen, Propylen, Vinyltoluol, Polystyrolmakromer und Gemischen hievon ausgewählt ist, und vorzugsweise unter n-Butylmethacrylat, Isobutylmethacrylat, 2-Ethylhexylmethacrylat, tert.Butylacrylat, tert.Butylmethacrylat, Methylmethacrylat und Gemischen hievon ausgewählt ist, und worin das Monomer B unter Acrylsäure, Methacrylsäure, N,N-Dimethylacrylamid, Dimethylaminoethylmethacrylat, quaternisiertem Dimethylaminoethylmethacrylat, Methacrylnitril, Methacrylamid, Maleinsäureanhydrid, Halbestern von Maleinsäureanhydrid, Itaconsäure, Acrylamid, Acrylatalkoholen, Hydroxyethylmethacrylat, Diallyldimethylammoniumchlorid, Vinylpyrrolidon, Vinylethern, Maleimiden, Vinylpyridin, Vinylimidazol, Styrolsulfonat und Gemischen hievon ausgewählt ist und vorzugsweise unter Acrylsäure, N,N-Dimethylacrylamid, Dimethylaminoethylmethacrylat, quaternisiertem Dimethylaminoethylmethacrylat, Vinylpyrrolidon und Gemischen hievon ausgewählt ist.

**14.** Haarpflegezusammensetzung nach einem der Ansprüche 11 bis 13, worin das Monomer C die Formel

$$X - \overset{\overset{O}{\|}}{C} - O - (CH_2)_q - (O)_p - Si(R_4)_{3-m}Zm$$

besitzt, vorzugsweise worin p den Wert 0 aufweist und q den Wert 3 besitzt, und am stärksten bevorzugt, worin m für 1 steht, r 250 beträgt, $R^4$ Alkyl bedeutet, $R^1$ Wasserstoff darstellt und $R^2$ Methyl ist.

**15.** Haarpflegezusammensetzung nach einem der Ansprüche 11 bis 14, worin das Silikon enthaltende Copolymer unter
Acrylsäure/n-Butylmethacrylat/Polydimethylsiloxan-Makromer - MG 20.000 (10/70/20);
N,N-Dimethylacrylamid/Isobutylmethacrylat/PDMS-Makromer - MG 20.000 (20/60/20);
Dimethylaminoethylmethacrylat/Isobutylmethacrylat/2-Ethylhexylmethacrylat/PDMS - MG 20.000 (25/40/15/20);
Dimethylaminoethylmethacrylat/Isobutylmethacrylat/PDMS - MG 20.000 (10/70/20);
quaternisiertem Dimethylaminoethylmethacrylat/Isobutylmethacrylat/PDMS - MG 20.000 (40/40/20);
Acrylsäure/Methylmethacrylat/PDMS - MG 20.000 (40/40/20);
Acrylsäure/Isopropylmethacrylat/PDMS - MG 20.000 (25/65/10);
N,N-Dimethylacrylamid/Methoxyethylmethacrylat/PDMS - MG 20.000 (60/25/15);
Dimethylacrylamid/PDMS-Makromer - MG 20.000 (80/20);
tert.Butylacrylat/tert.Butylmethacrylat/PDMS-Makromer - MG 10.000 (56/24/20);
tert.Butylacrylat/PDMS-Makromer - MG 10.000 (80/20);
tert.Butylacrylat/N,N-Dimethylacrylamid/PDMS-Makromer - MG 10.000 (70/10/20);
tert.Butylacrylat/Acrylsäure/PDMS-Makromer - MG 10.000 (75/5/20);
und Gemischen hievon ausgewählt ist.

**16.** Kosmetische Zusammensetzung nach einem der Ansprüche 1 bis 5, welche eine Haarpflegezusammensetzung ist, worin die Zusammensetzung:
(a) 80% bis 99,9% des Trägersystems, umfassend:
(A) 0,2% bis 5,0%, bezogen auf das Gewicht der Haarpflegezusammensetzung, von einem nichtionischen Celluloseether mit einer molaren Hydroxyethylsubstitution von 2,3% bis 3,7%, und welcher ferner mit einer $C_{16}$-Alkylgruppe in einer Menge von 0,40 Gew.-% bis 0,95 Gew.-% substituiert ist, worin die unsubstituierte Hydroxyethylcellulose ein mittleres Molekulargewicht von 300.000 bis 700.000 besitzt;
(B) 0,05% bis 3,0%, bezogen auf das Gewicht der Haarpflegezusammensetzung, von dem wasserunlöslichen grenzflächenaktiven Mittel, worin das grenzflächenaktive Mittel unter Stearamid-DEA, Kokosamid-MEA, Dimethylstearaminoxid, Glycerylmonooleat, Saccharosestearat, PEG-2-Stearamin, Ceteth-2, Glycerinstearatcitrat, hydriertem Ditalgdimethylammoniumchlorid, Poloxamer 181, hydriertem Talgdimethylbetain, hydriertem Talgamid-DEA, und Gemischen hievon ausgewählt ist;
(C) 0,05% bis 0,3% eines Chelatbildners, welcher unter Ethylendiamintetraessigsäure und den Salzen hievon, Zitronensäure und den Salzen hievon und Phosphorsäure und den Salzen hievon ausgewählt ist;
(D) 0,05% bis 1,0% von einem Verteilungshilfsmittel, welches unter Xanthangummi und Dextran mit einem Molekulargewicht von mehr als 1,000.000 ausgewählt ist; und
(b) 0,1% bis 20% der wirksamen kosmetischen Komponente enthält, welche eine Haarpflegekomponente ist, die unter
(A) einem Silikon enthaltenden Copolymer, welches unter
Acrylsäure/n-Butylmethacrylat/Polydimethylsiloxanmakromer - MG 20.000 (10/70/20);
N,N-Dimethylacrylamid/Isobutylmethacrylat/PDMS-Makromer - MG 20.000 (20/60/20);
Dimethylaminoethylmethacrylat/Isobutylmethacrylat/2-Ethylhexylmethacrylat/PDMS - MG 20.000 (25/40/15/20);

Dimethylaminoethylmethacrylat/Isobutylmethacrylat/PDMS - MG 20.000 (10/70/20);
quaternisiertem Dimethylaminoethylmethacrylat/Isobutylmethacrylat/PDMS - MG 20.000 (40/40/20);
Acrylsäure/Methylmethacrylat/PDMS - MG 20.000 (40/40/20);
Acrylsäure/Isopropylmethacrylat/PDMS - MG 20.000 (25/65/10);
N,N-Dimethylacrylamid/Methoxyethylmethacrylat/PDMS - MG 20.000 (60/25/15);
Dimethylacrylamid/PDMS-Makromer - MG 20.000 (80/20);
tert.Butylacrylat/tert.Butylmethacrylat/PDMS-Makromer - MG 10.000 (56/24/20);
tert.Butylacrylat/PDMS-Makromer - MG 10.000 (80/20);
tert.Butylacrylat/N,N-Dimethylacrylamid/PDMS-Makromer - MG 10.000 (70/10/20);
tert.Butylacrylat/Acrylsäure/PDMS-Makromer - MG 10.000 (75/5/20); und Gemischen hievon ausgewählt ist, und

(B) einem Konditionierungsmittel auf Silikonbasis ausgewählt ist, welches unter

einem Konditionierungsmittel, umfassend:

(a) 0,1% bis 2,5% eines Polydimethylsiloxankautschukes;
(b) 0,02% bis 0,7% pyrogene Kieselsäure, und
(c) 0,4% bis 18% eines flüchtigen Silikonträgers;

einem Konditionierungsmittel, umfassend:

(a) ein flüchtiges Silikonöl mit einer Viskosität von weniger als 10 cP,
(b) 0,5% bis 2,0% eines Silikonkautschukes mit einer Viskosität von mehr als 1,000.000 cP,
in Verhältnissen vom flüchtigen Öl zum Kautschuk von 85:15 bis 50:50; und

einem Konditionierungsmittel, umfassend:

(a) ein nicht-flüchtiges Silikonöl mit einer Viskosität von weniger als 100.000 cP,
(b) 0,5% bis 2,0% eines Silikonkautschukes mit einer Viskosität von mehr als 1,000.000 cP,
in Verhältnissen vom nicht-flüchtigen Öl zum Kautschuk von 60:40 bis 40:60;

ausgewählt ist,

worin die genannte Haarpflegezusammensetzung nicht mehr als 0,5% an wasserlöslichen grenzflächenaktiven Mitteln; nicht mehr als 1% an Fettalkoholmaterialien enthält; und worin die genannte Haarpflegezusammensetzung ein Fließverhalten zeigt, welches durch eine Scherspannung von 0 bis 50 Pa über einem Schergeschwindigkeitsbereich von 0,04 $s^{-1}$ bis 25 $s^{-1}$ gekennzeichnet ist.

**17.** Kosmetische Zusammensetzung nach Anspruch 1, welche eine haarkonditionierende Zusammensetzung ist, worin die Zusammensetzung:

(a) 80% bis 99,9% des Trägersystems, umfassend:

(A) 0,1% bis 10,0%, bezogen auf das Gewicht der haarkonditionierenden Zusammensetzung, von einem nichtionischen Celluloseether mit einem Grad an unter Methyl, Hydroxyethyl und Hydroxypropyl ausgewählter nichtionischer Substitution, welcher ausreichend ist, um diesen wasserlöslich zu machen, und welcher Celluloseether ferner mit einem langkettigen Alkylrest mit 10 bis 24 Kohlenstoffatomen in einer Menge substituiert ist, welche von 0,2 Gew.-% bis zu der Menge reicht, welche die Löslichkeit des genannten Celluloseethers in Wasser auf weniger als 1 Gew.-% verringert, und welcher vorzugsweise von 0,2% bis 5,0% einer Hydroxyethylcellulose gebildet wird, welche mit einem langkettigen Alkylrest mit 16 Kohlenstoffatomen in einer Menge von 0,50 Gew.-% bis 0,95 Gew.-% substituiert ist; wobei die molare Hydroxyethylsubstitution von 2,3 bis 3,7 beträgt; und das mittlere Molekulargewicht der unsubstituierten Cellulose von 300.000 bis 700.000 ist;

(B) 0,02% bis 10,0%, vorzugsweise 0,05% bis 3,0%, bezogen auf das Gewicht der haarkonditionierenden Zusammensetzung, von einem wasserunlöslichen grenzflächenaktiven Mittel mit einem Molekulargewicht von weniger als 20.000, welches vorzugsweise unter Stearamid-DEA, Kokosamid-MEA, Dimethylstearaminoxid, Glycerylmonooleat, Saccharosestearat, PEG-2-Stearamin, Ceteth-2, Glycerinstearatcitrat, Poloxamer 181, hydriertem Talgdimethylbetain, hydriertem Talgamid-DEA und Gemischen hievon ausgewählt ist, und welches am stärksten bevorzugt hydriertes Talgamid-DEA enthält; und

(b) 0,1% bis 20% der wirksamen kosmetischen Komponente enthält, welche eine Haarpflegekomponente ist, umfassend:

(A) 0,1% bis 18%, bezogen auf das Gewicht der haarkonditionierenden Zusammensetzung, von einem Konditionierungsmittel auf Silikonbasis, welches vorzugsweise unter einem flüchtigen Silikonöl mit einer Viskosität von weniger als 10 cP; einem nicht-flüchtigen Silikonöl mit einer Viskosität von weniger als 100.000 cP, einem Silikonkautschuk mit einer Viskosität von mehr als

1,000.000 cP ausgewählt ist, welcher vorzugsweise unter Polydimethylsiloxankautschuken und Polyphenylmethylsiloxankautschuken ausgewählt ist; und

(B) bis zu 1%, bezogen auf das Gewicht der haarkonditionierenden Zusammensetzung, von einem Fettalkohol, welcher vorzugsweise unter Stearylalkohol, Cetylalkohol, Myristylalkohol, Behenylalkohol, Laurylalkohol, Oleylalkohol und Gemischen hievon ausgewählt ist, und welcher am stärksten bevorzugt unter Cetylalkohol, Stearylalkohol und Gemischen hievon ausgewählt ist;

worin eine quaternäre Ammoniumverbindung mindestens einen Teil des wasserunlöslichen grenzflächenaktiven Mittels in einer Menge bis zu 2,5% der konditionierenden Zusammensetzung bildet.

**18.** Zusammensetzung nach Anspruch 17, worin das eine quaternäre Ammoniumverbindung umfassende Haarkonditionierungsmittel 0,5% bis 2% hydriertes Ditalgdimethylammoniumchlorid enthält.

**19.** Zusammensetzung nach Anspruch 17 oder 18, welche zusätzlich 0,05% bis 1,0% eines Chelatbildners umfaßt, welcher unter Ethylendiamintetraessigsäure und den Salzen hievon, Nitrilotriessigsäure und den Salzen hievon, Hydroxyethylendiamintriessigsäure und den Salzen hievon, Diethylentriaminpentaessigsäure und den Salzen hievon, Diethanolglycin und den Salzen hievon, Ethanoldiglycin und Salzen hievon, Zitronensäure und den Salzen hievon, Phosphorsäure und den Salzen hievon ausgewählt ist.

**20.** Zusammensetzung nach einem der Ansprüche 17 bis 19, worin das Konditionierungsmittel auf Silikonbasis unter einer Kombination aus einem nicht-flüchtigen Silikonöl mit einer Viskosität von weniger als 100.000 cP und 0,015% bis 9,0% eines Silikonkautschukes mit einer Viskosität von mehr als 1,000.000 cP in einem Verhältnis von nicht-flüchtigem Öl zu Kautschuk von 70:30 bis 30:70; und einer Kombination aus einem flüchtigen Silikonöl mit einer Viskosität von weniger als 10 cP und 0,015% bis 9,0% eines Silikonkautschukes mit einer Viskosität von mehr als 1,000.000 cP in einem Verhältnis von flüchtigem Öl zu Kautschuk von 90:10 bis 10:90 ausgewählt ist.

**21.** Zusammensetzung nach einem der Ansprüche 17 bis 20, welche bis zu 1,0% von einem Trimethylsilylamidomethicon umfaßt, das zumindest einen Teil des Konditionierungsmittels auf Silikonbasis bildet.

**22.** Zusammensetzung nach einem der Ansprüche 17 bis 21, worin das Stearamidopropyldimethylamin mindestens einen Teil der wasserunlöslichen grenzflächenaktiven Komponente in einer Menge bis zu 1% der Konditionierungszusammensetzung bildet.

**23.** Zusammensetzung nach einem der Ansprüche 17 bis 22, welche zusätzlich 0,1% bis 1,5% eines hydrolysierten tierischen Proteins umfaßt.

**24.** Kosmetische Zusammensetzung nach einem der Ansprüche 1 bis 5, welche eine haarkonditionierende Zusammensetzung ist, worin die Zusammensetzung:

(a) 80% bis 99,9% des Trägersystems, umfassend:

(A) 0,2% bis 5,0%, bezogen auf das Gewicht der haarkonditionierenden Zusammensetzung, von einem nichtionischen Hydroxyethylcelluloseether, welcher mit einem langkettigen Alkylrest mit 16 Kohlenstoffatomen in einer Menge von 0,50 Gew.-% bis 0,95 Gew.-% substituiert ist; mit einer molaren Hydroxyethylsubstitution von 2,3 bis 3,7; und einem mittleren Molekulargewicht der unsubstituierten Cellulose von 300.000 bis 700.000;

(B) 0,05% bis 3,0%, bezogen auf das Gewicht der haarkonditionierenden Zusammensetzung, von hydriertem Talgamid-DEA;

(C) 0,05% bis 0,3%, bezogen auf das Gewicht der haarkonditionierenden Zusammensetzung, von einem Chelatbildner, welcher unter Ethylendiamintetraessigsäure und den Salzen hievon; Zitronensäure und den Salzen hievon; und Gemischen hievon ausgewählt ist, worin das verträgliche Lösungsmittel Wasser ist; und

(b) 0,1% bis 20% einer wirksamen Haarpflegekomponente enthält, welche

(A) 0,5% bis 15%, bezogen auf das Gewicht der haarkonditionierenden Zusammensetzung, von einem Konditionierungsmittel auf Silikonbasis, welches unter einer Kombination aus einem flüchtigen Silikonöl mit einer Viskosität von weniger als 10 cP und 0,5% bis 2,0% eines Silikonkautschukes mit einer Viskosität von mehr als 1,000.000 cP in Verhältnissen vom flüchtigen Öl zum Kautschuk von 85:15 bis 50:50; und einer Kombination aus einem nicht-flüchtigen Silikonöl mit einer Viskosität von weniger als 100.000 cP und 0,5% bis 2,0% eines Silikonkautschukes mit einer Viskosität von mehr als 1,000.000 cP in Verhältnissen vom nicht-flüchtigen Öl zum

Kautschuk von 60:40 bis 40:60 ausgewählt ist; und
(B) 0,5% bis 2,0%, bezogen auf das Gewicht der haarkonditionierenden Zusammensetzung, von hydriertem Ditalgdimethylammoniumchlorid; und
(C) bis zu 1%, bezogen auf das Gewicht der haarkonditionierenden Zusammensetzung, von einem Fettalkohol, welcher unter Cetylalkohol, Stearylalkohol und Gemischen hievon ausgewählt ist,

umfaßt, worin die genannte haarkonditionierende Zusammensetzung nicht mehr als 0,5% an wasserlöslichen grenzflächenaktiven Mitteln enthält.

**25.** Verfahren zur Herstellung einer haarkonditionierenden Zusammensetzung nach einem der Ansprüche 17 bis 24, umfassend den Schritt des vorherigen Vermischens des Konditionierungsmittels auf Silikonbasis, der quaternären Ammoniumverbindung und mindestens einem Teil des Lösungsmittels vor dem Mischen mit den verbleibenden Komponenten.

## Revendications

**1.** Composition cosmétique, caractérisée en ce qu'elle comprend:
(a) de 80% à 100% d'un système véhicule qui comprend:
(A) de 0,1% à 10,0%, en poids de la composition cosmétique, d'un polymère non ionique hydrosoluble, modifié par des groupes hydrophobes, qui comprend une chaîne principale polymère hydrosoluble et des groupes hydrophobes choisis parmi les groupes alkyle, arylalkyle, alkylaryle en $C_8$-$C_{22}$, et des mélanges de ceux-ci; dans lequel le rapport de la partie hydrophile à la partie hydrophobe du polymère est de 10:1 à 1 000:1, qui est de préférence un éther de cellulose non ionique, choisi parmi les éthers méthyliques, hydroxyéthyliques et hydroxypropyliques, ayant un degré de substitution non ionique suffisant pour le rendre hydrosoluble, et qui est en outre substitué par un radical alkyle à chaîne longue possédant 10 à 24 atomes de carbone, en une quantité comprise entre 0,2% en poids et la quantité qui rend ledit éther de cellulose soluble dans l'eau à raison de moins de 1% en poids; et
(B) de 0,02% à 10,0%, en poids de la composition cosmétique, d'un tensioactif insoluble dans l'eau ayant une masse moléculaire inférieure à 20 000; et
(C) de 65% à 99%, en poids de la composition cosmétique, d'un solvant compatible; et
(b) de 0% à 20% d'un constituant cosmétique actif;

dans laquelle lesdites compositions cosmétiques ne comprennent pas plus de 1,0% de tensioactifs hydrosolubles et, mieux encore, dans laquelle le système véhicule fournit une rhéologie à la composition cosmétique qui est caractérisée par une contrainte de cisaillement de 0 à 50 pascals sur une gamme de vitesses de cisaillement de 0,04 $s^{-1}$ à 25 $s^{-1}$.

**2.** Composition selon la revendication 1, dans laquelle l'éther de cellulose non ionique constitue de 0,2% à 5,0% de la composition cosmétique, et de préférence dans laquelle l'éther de cellulose non ionique comprend le radical alkyle à chaîne longue fixé par une liaison éther.

**3.** Composition selon la revendication 1 ou 2, dans laquelle l'éther de cellulose non ionique est choisi parmi une hydroxypropylcellulose hydrosoluble substituée par un radical alkyle à chaîne longue possédant 10 à 24 atomes de carbone, en une quantité comprise entre 0,2% en poids et la quantité qui rend ladite hydroxypropylcellulose soluble dans l'eau à raison de moins de 1% en poids; et une hydroxyéthylcellulose hydrosoluble, qui possède de préférence une masse moléculaire de 50 000 à 700 000, qui est substituée par un radical alkyle à chaîne longue possédant 10 à 24 atomes de carbone, en une quantité comprise entre 0,2% en poids et la quantité qui rend ladite hydroxyéthylcellulose soluble dans l'eau à raison de moins de 1% en poids.

**4.** Composition selon l'une quelconque des revendications 1-3, dans laquelle l'hydroxyéthylcellulose hydrosoluble est substituée par un radical alkyle à chaîne longue possédant 16 atomes de carbone, en une quantité comprise entre 0,40% et 0,95% en poids ; la substitution molaire hydroxyéthyle est de 2,3 à 3,7; et la masse moléculaire moyenne de la cellulose non substituée est de 300 000 à 700 000.

**5.** Composition selon l'une quelconque des revendications 1-4, dans laquelle le tensioactif insoluble dans l'eau est présent en proportion de 0,05% à 3,0% et est choisi parmi la stéaramide DEA, la coprahamide MEA, l'oxyde de diméthylstéaramine, le monooléate de glycéryle, le stéarate de saccharose, la

PEG-2 stéaramine, le Ceteth-2, le stéarate citrate de glycérol, le chlorure de di(suif hydrogéné)-diméthyl-ammonium, le Poloxamer 181, la suif hydrogéné-diméthyl-bétaïne, la suif hydrogéné-amide DEA, et leurs mélanges.

6. Composition selon la revendication 3, qui comprend aussi de 0,3% à 5,0% d'une matière polymère hydrosoluble ayant une masse moléculaire supérieure à 20 000, qui est de préférence choisie parmi l'hydroxyéthylcellulose, l'hydroxypropylcellulose, l'hydroxypropylméthylcellulose, le polyéthylèneglycol, le polyacrylamide, le poly(acide acrylique), le poly(alcool vinylique), la polyvinylpyrrolidone, le dextran, la carboxyméthylcellulose, l'exsudat d'acacia, l'exsudat de ghatti, l'exsudat d'adragante, l'alginate de sodium, l'alginate de propylèneglycol, la carragénine sodique, les polysaccharides naturels, et leurs mélanges, et qui est tout particulièrement un polysaccharide naturel, choisi parmi la gomme de guar, la gomme de caroube, la gomme xanthane, et leurs mélanges.

7. Composition selon l'une quelconque des revendications 1-6, qui comprend aussi de 0,05% à 1,0%, en poids de la composition, d'un agent chélateur, qui est de préférence choisi parmi l'acide éthylènediamine-tétraacétique et ses sels, l'acide nitriloacétique et ses sels, l'acide hydroxyéthylènediamine-triacétique et ses sels, l'acide diéthylènetriamine-pentaacétique et ses sels, la diéthanolglycine et ses sels, l'éthanoldiglycine et ses sels, l'acide citrique et ses sels, l'acide phosphorique et ses sels.

8. Composition selon l'une quelconque des revendications 1-7, dans laquelle de 0,02% à 2,5% du polymère hydrosoluble sont choisis parmi les matières polymères hydrosolubles ayant une masse moléculaire supérieure à 1 000 000, et les matières polymères hydrosolubles ayant un fort caractère ionique.

9. Composition cosmétique selon l'une quelconque des revendications 1-8, qui est une composition de soin des cheveux qui ne comprend de préférence pas plus de 1% de substances alcools gras, dans laquelle ledit constituant cosmétique actif comprend un constituant de soin des cheveux actif, qui est de préférence choisi parmi les agents de conditionnement, les auxiliaires antipelliculaires, les agents favorisant la pousse des cheveux, les parfums, les colorants, les pigments, les polymères de tenue de la coiffure, et leurs mélanges, et qui est, mieux encore, choisi parmi une huile de silicone volatile ayant une viscosité inférieure à 10 centipoises; une huile de silicone non volatile ayant une viscosité inférieure à 100 000 cP; une gomme de silicone ayant une viscosité supérieure à 1 000 000 cP, qui est de préférence choisie parmi les gommes de polydiméthylsiloxane et les gommes de polyphénylméthylsiloxane; et leurs mélanges.

10. Composition selon la revendication 9, dans laquelle le constituant actif de soin des cheveux comprend de 0,01% à 10% d'un polymère silicone rigide ayant une viscosité complexe d'au moins 2 x $10^5$ poises, qui est de préférence choisi parmi les gommes de siloxane à substitution organique, les élastomères de silicone, les gommes de polydiméthylsiloxane à renfort de charges, les siloxanes à renfort de résine et les polymères de siloxane réticulés; et un véhicule volatil pour le polymère silicone rigide, qui est de préférence une silicone cyclique contenant de 3 à 7 atomes de silicium.

11. Composition selon la revendication 9, dans laquelle le constituant actif de soin des cheveux comprend de 0,1% à 10,0% d'un copolymère ayant une chaîne principale polymère vinylique sur laquelle sont greffés des groupements polymères siloxane monovalents, ledit copolymère comprenant des monomères C et des constituants choisis parmi les monomères A, les monomères B et des mélanges de ceux-ci, où:
A est au moins un monomère vinylique polymérisable par voie radicalaire, la quantité, en poids, de monomère A, lorsqu'on l'utilise, allant jusqu'à 98%, de préférence de 5% à 98%, en poids, par rapport au poids total de tous les monomères dans ledit copolymère;
B est au moins un monomère de renfort copolymérisable avec A, la quantité, en poids, de monomère B, lorsqu'on l'utilise, allant jusqu'à 98% du poids total de tous les monomères dans ledit copolymère, ledit monomère B étant choisi parmi les monomères et les macromères polaires; et
C est un monomère polymère ayant une masse moléculaire de 1 000 à 50 000 et répondant à la formule générale

$$X(Y)_nSi(R)_{3-m}(Z)_m$$

dans laquelle

X est un groupe vinyle copolymérisable avec les monomères A et B;

Y est un groupe de liaison divalent

R est un atome d'hydrogène ou un groupe alkyle inférieur, aryle ou alcoxy

Z est un groupement polymère siloxane monovalent ayant une masse moléculaire moyenne en nombre d'au moins 500, est essentiellement non réactif dans les conditions de la copolymérisation et est pendant sur ladite chaîne principale polymère vinylique après la polymérisation

n est égal à 0 ou 1

m est un nombre entier de 1 à 3

dans laquelle C constitue de 0,01% à 50%, de préférence de 0,1% à 50%, du copolymère.

**12.** Composition selon la revendication 11, dans laquelle le constituant actif de traitement des cheveux comprend un monomère vinylique (A) polymérisable par voie radicalaire, lipophile, de faible polarité, un monomère polaire hydrophile (B) qui est copolymérisable avec A, et un macromère contenant une silicone, ayant une masse moléculaire moyenne en poids de 1 000 à 50 000, à base d'un polydiméthylsiloxane choisi parmi

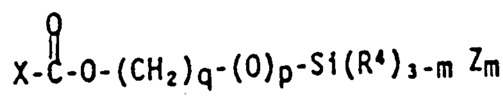

$X\text{-}Si(R^4)_{3-m}\ Z_m$

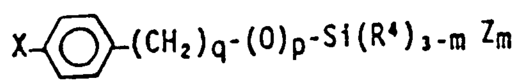

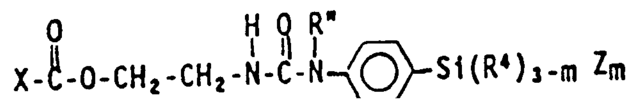

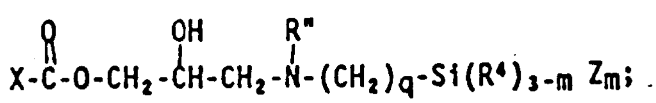 et

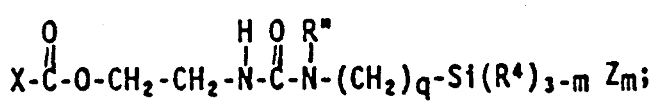

où m est égal à 1, 2 ou 3; p est égal à 0 ou 1; R" est un groupe alkyle ou un atome d'hydrogène; q est un nombre entier de 2 à 6; s est un nombre entier de 0 à 2; X est

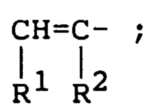

$R^1$ est un atome d'hydrogène ou -COOH; $R^2$ est un atome d'hydrogène ou un groupe méthyle ou $-CH_2COOH$; Z est

$$R^4-(-\overset{\displaystyle CH_3}{\underset{\displaystyle CH_3}{Si}}-O-)_r \;;$$

$R^4$ est un groupe alkyle, alcoxy, alkylamino, aryle ou hydroxyle; et r est un nombre entier de 5 à 700.

**13.** Composition de soin des cheveux selon la revendication 11 ou 12, dans laquelle le monomère A est choisi parmi les esters d'acide acrylique d'alcools en $C_1$-$C_{18}$, les esters d'acide méthacrylique d'alcools en $C_1$-$C_{18}$, le styrène, l'acétate de vinyle, le chlorure de vinyle, le chlorure de vinylidène, l'acrylonitrile, l'$\alpha$-méthylstyrène, le t-butylstyrène, le butadiène, le cyclohexadiène, l'éthylène, le propylène, le vinyltoluène, un macromère polystyrène et des mélanges de ceux-ci; et est de préférence choisi parmi le méthacrylate de n-butyle, le méthacrylate d'isobutyle, le méthacrylate de 2-éthylhexyle, l'acrylate de t-butyle, le méthacrylate de t-butyle, le méthacrylate de méthyle et des mélanges de ceux-ci, et dans laquelle le monomère B est choisi parmi l'acide acrylique, l'acide méthacrylique, le N,N-diméthylacrylamide, le méthacrylate de diméthylaminoéthyle, le méthacrylate de diméthylaminoéthyle quaternisé, le méthacrylonitrile, le méthacrylamide, l'anhydride maléique, les hémiesters d'anhydride maléique, l'acide itaconique, l'acrylamide, les acrylate-alcools, le méthacrylate d'hydroxyéthyle, le chlorure de diallyldiméthylammonium, la vinylpyrrolidone, les éthers vinyliques, les maléimides, la vinylpyridine, le vinylimidazole, le styrènesulfonate, et des mélanges de ceux-ci, et est de préférence choisi parmi l'acide acrylique, le N,N-diméthylacrylamide, le méthacrylate de diméthylaminoéthyle, le méthacrylate de diméthylaminoéthyle quaternisé, la vinylpyrrolidone, et des mélanges de ceux-ci.

**14.** Composition de soin des cheveux selon l'une quelconque des revendications 11-13, dans laquelle le monomère C répond à la formule

$$X - \overset{\displaystyle O}{\overset{\|}{C}} - O - (CH_2)_q - (O)_p - Si(R^4)_{3-m}Z_m$$

de préférence dans laquelle p = 0 et q = 3, et mieux encore dans laquelle m est égal à 1, r est égal à 250, $R^4$ est un groupe alkyle, $R^1$ est un atome d'hydrogène et $R^2$ est un groupe méthyle.

**15.** Composition de soin des cheveux selon l'une quelconque des revendications 11-14, dans laquelle le copolymère contenant une silicone est choisi parmi
un macromère acide acrylique/méthacrylate de n-butyle/polydiméthylsiloxane - Mp 20 000 (10/70/20);
un macromère N,N-diméthylacrylamide/méthacrylate d'isobutyle/PDMS - Mp 20 000 (20/60/20);
un méthacrylate de diméthylaminoéthyle/méthacrylate d'isobutyle/méthacrylate de 2-éthylhexyle/PDMS - Mp 20 000 (25/40/15/20);
un méthacrylate de diméthylaminoéthyle/méthacrylate d'isobutyle/PDMS - Mp 20 000 (10/70/20);
un méthacrylate de diméthylaminoéthyle quaternisé/méthacrylate d'isobutyle/PDMS - Mp 20 000 (40/40/20);
un acide acrylique/méthacrylate de méthyle/PDMS - Mp 20 000 (40/40/20);
un acide acrylique/méthacrylate d'isopropyle/PDMS - Mp 20 000 (25/65/10);
un N,N-diméthylacrylamide/méthacrylate de méthoxyéthyle/PDMS - Mp 20 000 (60/25/15);
un macromère diméthylacrylamide/PDMS - Mp 20 000 (80/20);
un macromère acrylate de t-butyle/méthacrylate de t-butyle/PDMS - Mp 10 000 (56/24/20);
un macromère acrylate de t-butyle/PDMS - Mp 10 000 (80/20);
un macromère acrylate de t-butyle/N,N-diméthylacrylamide/PDMS - Mp 10 000 (70/10/20);
un macromère acrylate de t-butyle/acide acrylique/PDMS - Mp 10 000 (75/5/20);
et des mélanges de ceux-ci.

**16.** Composition cosmétique selon l'une quelconque des revendications 1 à 5, qui est une composition de soin des cheveux, dans laquelle la composition comprend:
(a) de 80% à 99,9% du système véhicule qui comprend:

(A) de 0,2% à 5,0%, en poids de la composition de soin des cheveux, d'un éther de cellulose non ionique ayant un taux molaire de substitution hydroxyéthyle de 2,3% à 3,7% et étant en outre substitué par un groupe alkyle en $C_{16}$ à raison de 0,40% à 0,95% en poids, l'hydroxyéthylcellulose non substituée possédant une masse moléculaire moyenne de 300 000 à 700 000;

(B) de 0,05% à 3,0%, en poids de la composition de soin des cheveux, du tensioactif insoluble dans l'eau, le tensioactif étant choisi parmi la stéaramide DEA, la coprah-amide MEA, l'oxyde de diméthylstéaramine, le monooléate de glycéryle, le stéarate de saccharose, la PEG-2 stéaramine, le Ceteth-2, le stéarate citrate de glycérol, le chlorure de di(suif hydrogéné)-diméthyl-ammonium, le Poloxamer 181, la suif hydrogéné-diméthyl-bétaïne, la suif hydrogéné-amide DEA, et leurs mélanges;

(C) de 0,05% à 0,3% d'un agent chélateur, qui est choisi parmi l'acide éthylènediaminetétraacétique et ses sels, l'acide citrique et ses sels, et l'acide phosphorique et ses sels;

(D) de 0,05% à 1,0% d'un auxiliaire de distribution, qui est choisi parmi la gomme xanthane et le dextran ayant une masse moléculaire supérieure à 1 000 000; et

(b) de 0,1% à 20% du constituant cosmétique actif qui est un constituant de soin des cheveux, choisi parmi:

(A) un copolymère contenant une silicone, choisi parmi
un macromère acide acrylique/méthacrylate de n-butyle/polydiméthylsiloxane - Mp 20 000 (10/70/20);
un macromère N,N-diméthylacrylamide/méthacrylate d'isobutyle/PDMS - Mp 20 000 (20/60/20);
un méthacrylate de diméthylaminoéthyle/méthacrylate d'isobutyle/méthacrylate de 2-éthylhexyle/PDMS - Mp 20 000 (25/40/15/20);
un méthacrylate de diméthylaminoéthyle/méthacrylate d'isobutyle/PDMS - Mp 20 000 (10/70/20);
un méthacrylate de diméthylaminoéthyle quaternisé/méthacrylate d'isobutyle/PDMS - Mp 20 000 (40/40/20);
un acide acrylique/méthacrylate de méthyle/PDMS - Mp 20 000 (40/40/20);
un acide acrylique/méthacrylate d'isopropyle/PDMS - Mp 20 000 (25/65/10);
un N,N-diméthylacrylamide/méthacrylate de méthoxyéthyle/PDMS - Mp 20 000 (60/25/15);
un macromère diméthylacrylamide/PDMS - Mp 20 000 (80/20);
un macromère acrylate de t-butyle/méthacrylate de t-butyle/PDMS - Mp 10 000 (56/24/20);
un macromère acrylate de t-butyle/PDMS - Mp 10 000 (80/20);
un macromère acrylate de t-butyle/N,N-diméthylacrylamide/PDMS - Mp 10 000 (70/10/20);
un macromère acrylate de t-butyle/acide acrylique/PDMS - Mp 10 000 (75/5/20); et des mélanges de ceux-ci; et

(B) un agent de conditionnement silicone, qui est choisi parmi
un agent de conditionnement comprenant:
(a) de 0,1% à 2,5% d'une gomme de polydiméthylsiloxane;
(b) de 0,02% à 0,7% de fumée de silice; et
(c) de 0,4% à 18% d'un véhicule silicone volatil;
un agent de conditionnement comprenant:
(a) une huile de silicone volatile ayant une viscosité inférieure à 10 centipoises;
(b) de 0,5% à 2,0% d'une gomme de silicone ayant une viscosité supérieure à 1 000 000 centipoises;
dans des rapports de l'huile volatile à la gomme de 85:15 à 50:50; et
un agent de conditionnement comprenant:
(a) une huile de silicone non volatile ayant une viscosité inférieure à 100 000 centipoises;
(b) de 0,5% à 2,0% d'une gomme de silicone ayant une viscosité supérieure à 1 000 000 centipoises;
dans des rapports de l'huile non volatile à la gomme de 60:40 à 40:60,

dans laquelle ladite composition de soin des cheveux ne comprend pas plus de 0,5% de tensioactifs hydrosolubles; pas plus de 1% de substances alcools gras; et dans laquelle ladite composition de soin des cheveux possède une rhéologie qui est caractérisée par une contrainte de cisaillement de 0 à 50 pascals sur une gamme de vitesses de cisaillement de 0,04 $s^{-1}$ à 25 $s^{-1}$.

**17.** Composition cosmétique selon la revendication 1, qui est une composition de conditionnement des cheveux, dans laquelle la composition comprend:

(a) de 80% à 99,9% du système véhicule qui comprend:

(A) de 0,1% à 10,0%, en poids de la composition de conditionnement des cheveux, d'un éther de cellulose non ionique, choisi parmi les éthers méthyliques, hydroxyéthyliques et hydroxypropyliques, ayant un degré de substitution non ionique suffisant pour le rendre hydrosoluble, qui est en outre substitué par un radical alkyle à chaîne longue possédant 10 à 24 atomes de carbone, en une quantité comprise entre 0,2% en poids et la quantité qui rend ledit éther de cellulose soluble dans l'eau à raison de moins de 1% en poids, et qui est de préférence de 0,2% à 5,0% d'une hydroxyéthylcellulose substituée par un radical alkyle à chaîne longue possédant 16 atomes de carbone, à raison de 0,50% à 0,95% en poids; le taux molaire de substitution hydroxyéthyle est de 2,3 à 3,7; et la masse moléculaire moyenne de la cellulose non substituée est de 300 000 à 700 000;

(B) de 0,02% à 10,0%, de préférence de 0,05% à 3,0%, en poids de la composition de conditionnement des cheveux, d'un tensioactif insoluble dans l'eau ayant une masse moléculaire inférieure à 20 000, qui est de préférence choisi parmi la stéaramide DEA, la coprah-amide MEA, l'oxyde de diméthylstéaramine, le monooléate de glycéryle, le stéarate de saccharose, la PEG-2 stéaramine, le Ceteth-2, le stéarate citrate de glycérol, le Poloxamer 181, la suif hydrogéné-diméthyl-bétaïne, la suif hydrogéné-amide DEA, et leurs mélanges, et qui comprend tout particulièrement de la suif hydrogéné-amide DEA; et

(b) de 0,1% à 20% du constituant cosmétique actif qui est un constituant de soin des cheveux comprenant:

(A) de 0,1% à 18%, en poids de la composition de conditionnement des cheveux, d'un agent de conditionnement silicone qui est de préférence choisi parmi une huile de silicone volatile ayant une viscosité inférieure à 10 centipoises; une huile de silicone non volatile ayant une viscosité inférieure à 100 000 centipoises; une gomme de silicone ayant une viscosité supérieure à 1 000 000 centipoises, qui est de préférence choisie parmi les gommes de polydiméthylsiloxane et les gommes de polyphénylméthylsiloxane; et

(B) jusqu'à 1%, en poids de la composition de conditionnement des cheveux, d'un alcool gras qui est de préférence choisi parmi l'alcool stéarylique, l'alcool cétylique, l'alcool myristylique, l'alcool béhénylique, l'alcool laurylique, l'alcool oléylique, et des mélanges de ceux-ci, et qui est tout particulièrement choisi parmi l'alcool cétylique, l'alcool stéarylique, et des mélanges de ceux-ci;

dans laquelle un composé d'ammonium quaternaire constitue au moins une partie du tensioactif insoluble dans l'eau, dans une proportion allant jusqu'à 2,5% de la composition de conditionnement.

**18.** Composition selon la revendication 17, dans laquelle l'agent de conditionnement des cheveux à base de composé d'ammonium quaternaire comprend de 0,5% à 2% de chlorure de di-suif (hydrogéné) diméthylammonium.

**19.** Composition selon la revendication 17 ou 18, comprenant aussi de 0,05% à 1,0% d'un agent chélateur qui est choisi parmi l'acide éthylènediamine-tétraacétique et ses sels, l'acide nitrilotriacétique et ses sels, l'acide hydroxyéthylènediamine-triacétique et ses sels, l'acide diéthylènetriamine-pentaacétique et ses sels, la diéthanolglycine et ses sels, l'éthanoldiglycine et ses sels, l'acide citrique et ses sels, l'acide phosphorique et ses sels.

**20.** Composition selon l'une quelconque des revendications 17-19, dans laquelle l'agent de conditionnement silicone est choisi parmi une combinaison d'une huile de silicone non volatile ayant une viscosité inférieure à 100 000 cP, et de 0,015% à 9,0% d'une gomme de silicone ayant une viscosité supérieure à 1 000 000 cP, dans un rapport de l'huile non volatile à la gomme de 70:30 à 30:70; et une combinaison d'une huile de silicone volatile ayant une viscosité inférieure à 10 cP et de 0,015% à 9,0% d'une gomme de silicone ayant une viscosité supérieure à 1 000 000 cP, dans un rapport de l'huile volatile à la gomme de 90:10 à 10:90.

**21.** Composition selon l'une quelconque des revendications 17-20, qui comprend jusqu'à 1,0% d'une triméthylsilylamodiméthicone, au moins comme partie de l'agent de conditionnement silicone.

**22.** Composition selon l'une quelconque des revendications 17-21, dans laquelle une stéaramidopropyldiméthylamine constitue au moins une partie du constituant tensioactif insoluble dans l'eau, dans une proportion allant jusqu'à 1% de la composition de conditionnement.

**23.** Composition selon l'une quelconque des revendications 17-22, qui comprend aussi de 0,1% à 1,5% d'une protéine animale hydrolysée.

**24.** Composition cosmétique selon l'une quelconque des revendications 1 à 5, qui est une composition de conditionnement des cheveux, dans laquelle la composition comprend:

(a) de 80% à 99,9% du système véhicule qui comprend:

(A) de 0,2% à 5,0%, en poids de la composition de conditionnement des cheveux, d'un éther d'hydroxyéthylcellulose non ionique substitué par un radical alkyle à chaîne longue comportant 16 atomes de carbone, dans une proportion comprise entre 0,50% et 0,95% en poids; un taux molaire de substitution hydroxyéthyle de 2,3 à 3,7; et une masse moléculaire moyenne de la cellulose non substituée de 300 000 à 700 000;

(B) de 0,05% à 3,0%, en poids de la composition de conditionnement des cheveux, de suif hydrogénéamide DEA;

(C) de 0,05% à 0,3%, en poids de la composition de conditionnement des cheveux, d'un agent chélateur choisi parmi l'acide éthylènediamine-tétraacétique et ses sels; l'acide citrique et ses sels; et des mélanges de ceux-ci; où le solvant compatible est de l'eau; et

(b) de 0,1% à 20% d'un constituant actif de soin des cheveux comprenant:

(A) de 0,5% à 15%, en poids de la composition de conditionnement des cheveux, d'un agent de conditionnement silicone qui est choisi parmi une combinaison d'une huile de silicone volatile ayant une viscosité inférieure à 10 cP et de 0,5% à 2,0% d'une gomme de silicone ayant une viscosité supérieure à 1 000 000 cP, dans des rapports de l'huile volatile à la gomme de 85:15 à 50:50; et une combinaison d'une huile de silicone non volatile ayant une viscosité inférieure à 100 000 cP, et de 0,5% à 2,0% d'une gomme de silicone ayant une viscosité supérieure à 1 000 000 cP, dans des rapports de l'huile non volatile à la gomme de 60:40 à 40:60; et

(B) de 0,5% à 2,0%, en poids de la composition de conditionnement des cheveux, de chlorure de disuif (hydrogéné) diméthylammonium; et

(C) jusqu'à 1%, en poids de la composition de conditionnement des cheveux, d'un alcool gras choisi parmi l'alcool cétylique, l'alcool stéarylique, et des mélanges de ceux-ci;

dans laquelle ladite composition de conditionnement des cheveux ne comprend pas plus de 0,5% de tensioactifs hydrosolubles.

**25.** Procédé de fabrication d'une composition de conditionnement des cheveux selon l'une quelconque des revendications 17-24, comprenant l'étape qui consiste à mélanger au préalable l'agent de conditionnement silicone, le composé d'ammonium quaternaire et au moins une partie du solvant avant d'effectuer le mélange avec le reste des constituants.